# EUROPEAN PATENT SPECIFICATION

(11) **EP 2 635 104 B1**
(45) Date of publication and mention of the grant of the patent: **11.01.2017**
(21) Application number: 11781777.5
(22) Date of filing: 03.11.2011
(51) Int. Cl.: A01H 5/00, C12N 15/82, C07K 14/415

(54) **STRESS-RESISTANT PLANTS AND THEIR PRODUCTION**
STRESSRESISTENTE PFLANZEN UND IHRE HERSTELLUNG
PLANTES RÉSISTANTES AU STRESS ET LEUR PRODUCTION

(30) Priority: 04.11.2010 US 410074 P
(43) Date of publication of application: 11.09.2013
(73) Proprietor: Institut National de la Recherche Agronomique (INRA), 75338 Paris Cedex 07 (FR); Centre De Cooperation International En Recherche Agronomique (CIRAD), 34398 Montpellier (FR); UNIVERSITE DE MONTPELLIER, 34090 Montpellier (FR)
(72) Inventor: GANTET, Pascal, F-34830 Jacou (FR); GUIDERDONI, Emmanuel, F-34150 Aniane (FR); KHONG, Ngangiang, F-34090 Montpellier (FR); MOREL, Jean-Benoit, F-34090 Montpellier (FR)
(74) Representative: Becker, Philippe
(86) International application number: PCT/EP2011/069367
(87) International publication number: WO 2012/059559

(56) References cited:
- WO-A1-02/33091
- WO-A2-2007/113237
- E. SEO ET AL: "Crosstalk between Cold Response and Flowering in Arabidopsis Is Mediated through the Flowering-Time Gene SOC1 and Its Upstream Negative Regulator FLC", THE PLANT CELL ONLINE, vol. 21, no. 10, 1 October 2009 (2009-10-01), pages 3185-3197, XP55015943, ISSN: 1040-4651, DOI: 10.1105/tpc.108.063883
- S. LEE ET AL: "Further Characterization of a Rice AGL12 Group MADS-Box Gene, OsMADS26", PLANT PHYSIOLOGY, vol. 147, no. 1, 1 January 2008 (2008-01-01), pages 156-168, XP55015874, ISSN: 0032-0889, DOI: 10.1104/pp.107.114256 cited in the application
- DATABASE EMBL [Online] 27 November 2007 (2007-11-27), "Triticum aestivum mRNA for MIKC-type MADS-box transcription factor WM12 (WM12 gene)", XP002666800, retrieved from EBI accession no. EM_PL:AM502878 Database accession no. AM502878
- DATABASE Geneseq [Online] 30 April 2009 (2009-04-30), "Zea mays derived protein encoding gene SEQ ID 738.", XP002666801, retrieved from EBI accession no. GSN:AWI29215 Database accession no. AWI29215
- YINBO GAN ET AL: "Nutritional regulation of ANR1 and other root-expressed MADS-box genes in Arabidopsis thaliana", PLANTA ; AN INTERNATIONAL JOURNAL OF PLANT BIOLOGY, SPRINGER, BERLIN, DE, vol. 222, no. 4, 15 July 2005 (2005-07-15) , pages 730-742, XP019427438, ISSN: 1432-2048, DOI: 10.1007/S00425-005-0020-3
- TAO ZHAO ET AL: "Characterization and expression of 42 MADS-box genes in wheat (Triticum aestivum L.)", MOLECULAR GENETICS AND GENOMICS, SPRINGER, BERLIN, DE, vol. 276, no. 4, 21 July 2006 (2006-07-21) , pages 334-350, XP019443488, ISSN: 1617-4623, DOI: 10.1007/S00438-006-0121-0

## Description

### FIELD OF THE INVENTION

The present invention relates to plant genes involved in negative regulation of resistance to biotic and/or abiotic stress and uses thereof. More particularly, the present invention relates to plants comprising an inactivated MADS-box gene function, and having increased resistance to biotic and/or abiotic stress. The invention also relates to methods for producing modified plants having increased resistance to fungal, bacterial pathogens and/or to drought stress. In particular, the invention relates to methods for producing plants with inactivated MADS26 gene, or an ortholog thereof, and exhibiting resistance to biotic and/or abiotic stress.

### BACKGROUND OF THE INVENTION

Crop plants are continuously confronted with diverse pathogens. In particular, infection of crop plants with bacteria and fungi can have a devastating impact on agriculture due to loss of yield and contamination of plants with toxins. Other factors that cause drastic yield reduction in most crops are abiotic stress factors such as drought, salinity, heavy metals and temperature.

According to FAO estimates, diseases, insects and weeds cause as much as 25% yield losses annually in cereal crops (Khush, 2005). For example, in China alone, it is estimated that 1 million hectares are lost annually because of blast disease (Khush and Jena 2009). Between 1987 and 1996, fungicides represented, for example, up to 20 and 30% of the culture costs in China ($46 Million) and Japan ($461 Million) respectively.

To meet the increasing demand on the world food supply, it will be necessary to produce up to 40% more rice by 2030 (Khush 2005). This will have to be on a reduced sowing area due to urbanization and increasing environmental pollution. For example, the sowing area in China decreased by 8 million hectares between 1996 and 2007.

Improvement of yield per plant is not the only way to achieve this goal; reduction of losses by biotic and abiotic stress is also a solution.

One of the most devastating fungal diseases is a blast disease, which is caused by the ascomycete Magnaporthe oryzae, also known as rice blast fungus. Members of the M. grisea/M.oryzae complex (containing at least two biological species: M. grisea and M. oryzae) are extremely effective plant pathogens as they can reproduce both sexually and asexually to produce specialized infectious structures known as appressoria that infect aerial tissues and hyphae that can infect root tissues. Magnaporthe fungi can also infect a number of other agriculturally important cereals including wheat, rye, barley, and pearl millet causing diseases called blast disease or blight disease. Other plant fungal pathogens of economic importance include species fungal pathogens are selected from *Puccinia, Aspergillus, Ustilago, Septoria, Erisyphe, Rhizoctonia* and *Fusarium* species. Fusarium contamination in cereals (e.g., barley or wheat) can result in head blight disease. For example, the total losses in the US of barley and wheat crops between 1991 and 1996 have been estimated at $3 billion (Brewing Microbiology, 3rd edition. Priest and Campbell, ISBN 0-306-47288-0).

Other devastating for agriculture plant pathogens are bacterial pathogens from *Xanthomonas, Ralstonia, Erwinia, Pectobacterium, Pantoea, Agrobacterium, Pseudomonas, Burkholderia, Acidovorax, Clavibacter, Streptomyces, Xylella, Spiroplasma* and *Phytoplasma* species. Plant pathogenic bacteria cause many different kinds of symptoms that include galls and overgrowths, wilts, leaf spots, specks and blights, soft rots, as well as scabs and cankers. Some plant pathogenic bacteria produce toxins or inject special proteins that lead to host cell death or produce enzymes that break down key structural components of plant cells. An example is the production of enzymes by soft-rotting bacteria that degrade the pectin layer that holds plant cells together. Still others, such as *Ralstonia* spp., colonize the water-conducting xylem vessels causing the plants to wilt and die. *Agrobacterium* species even have the ability to genetically modify or transform their hosts and bring about the formation of cancer-like overgrowths called crown gall. Bacterial diseases in plants are difficult to control. Emphasis is on preventing the spread of the bacteria rather than on curing the plant.

Cultural practices can either eliminate or reduce sources of bacterial contamination, such as crop rotation to reduce over-wintering. However, the most important control procedure is ensured by genetic host resistance providing resistant varieties, cultivars, or hybrids.

Pathogen infection of crop plants can have a devastating impact on agriculture due to loss of yield and contamination of plants with toxins. Currently, outbreaks of blast disease are controlled by applying expensive and toxic fungicidal chemical treatments using for example probenazole, tricyclazole, pyroquilon and phthalide, or by burning infected crops. These methods are only partially successful since the plant pathogens are able to develop resistance to chemical treatments.

To reduce the amount of pesticides used, plant breeders and geneticists have been trying to identify disease resistance loci and exploit the plant's natural defense mechanism against pathogen attack. Plants can recognize certain pathogens and activate defense in the form of the resistance response that may result in limitation or stopping of pathogen growth. Many resistance (R) genes, which confer resistance to various plant species against a wide range of pathogens, have been identified. However, most of these R genes are usually not durable since pathogens can easily breakdown this type of resistance.

Consequently, there exists a high demand for novel efficient methods for controlling plant diseases, as well as for producing plants of interest with increased resistance to biotic and abiotic stress.

### SUMMARY OF THE INVENTION

The present invention provides novel and efficient methods for producing plants resistant to biotic and abiotic stress. Surprisingly, the inventors have discovered that mutant plants with a defective MADS-box gene are resistant to plant diseases. In particular, the inventors have demonstrated that MADS26 gene is a negative regulator of biotic stress response, and that plants with a defective MADS26 gene are resistant to fungal and bacterial pathogens while plants over-expressing the MADS26 gene are more susceptible to plant diseases. Moreover, the inventors have shown that inhibiting MADS26 gene expression increases plant resistance to drought stress. To our knowledge, this is the first example of regulation of biotic and abiotic resistance in plants by a transcription factor of the MADS-box family. In addition, the inventors have identified orthologs of MADS26 in various plants, as well as other members of the MADS-box gene family, thus extending the application of the invention to different cultures and modifications.
An object of this invention therefore relates to plants comprising a defective MADS26 function. As will be discussed, said plants exhibit an increased or improved resistance to biotic and/or abiotic stress. Preferably, said plants are monocots. More preferably, said plants are cereals selected from the Poaceae family (e.g., rice, wheat, barley, oat, rye, sorghum or maize).

The invention more particularly relates to plants having a defective MADS26 function and exhibiting an increased resistance to biotic and/or abiotic stress.

Another particular object of this invention relates to plants comprising a defective MADS-box gene and exhibiting an increased resistance to biotic and/or abiotic stress.

A further object of this invention relates to seeds of plants of the invention, or to plants, or descendents of plants grown or otherwise derived from said seeds.

A further object of the invention relates to a method for producing plants having increased resistance to biotic and/or abiotic stress, wherein the method comprises the following steps:
(a) inactivation of a MADS26 gene or protein, or an ortholog thereof, in a plant cell;
(b) optionally, selection of plant cells of step (a) with inactivated MADS26 function;
(c) regeneration of plants from cells of step (a) or (b); and
(d) optionally, selection of a plant of (c) with increased resistance to and biotic and/or abiotic stress, said plant having a defective MADS26 gene or protein, or an ortholog thereof.

As will be further disclosed in the present application, the MADS-box transcription factor function may be rendered defective by various techniques such as, for example, by inactivation of the gene (or RNA), inactivation of the protein, or inactivation of the transcription or translation thereof. Inactivation may be accomplished by, e.g., deletion, insertion and/or substitution of one or more nucleotides, site-specific mutagenesis, ethyl methanesulfonate (EMS) mutagenesis, targeting induced local lesions in genomes (TILLING), knock-out techniques, or gene silencing using, e.g., RNA interference, ribozymes, antisense, aptamers, and the like. The MADS-box function may also be rendered defective by altering the activity of the MADS-box protein, either by altering the structure of the protein, or by expressing in the cell a ligand of the protein, or an inhibitor thereof, for instance.

The invention also relates to a method for conferring or increasing resistance to biotic and/or abiotic stress to a plant, comprising a step of inhibiting, permanently or transiently, a MADS26 function in said plant, e.g., by inhibiting the expression of the MADS26 gene(s) in said plant.
Another object of this invention relates to an RNAi, as disclosed in the claims, that inhibits the expression (e.g., transcription or translation) of a MADS26 gene.

Another object of the invention relates to the use of such nucleic acid for increasing resistance of plants or plant cells to biotic and/or abiotic stress.

A further object of the invention relates to plants transformed with a vector comprising such an RNAi that inhibits the expression of a MADS26 gene.

The invention is applicable to produce cereals having increased resistance to biotic and/or abiotic stress, and is particularly suited to produce resistant wheat, rice, barley, oat, rye, sorghum or maize.

### BRIEF DESCRIPTION OF THE DRAWINGS

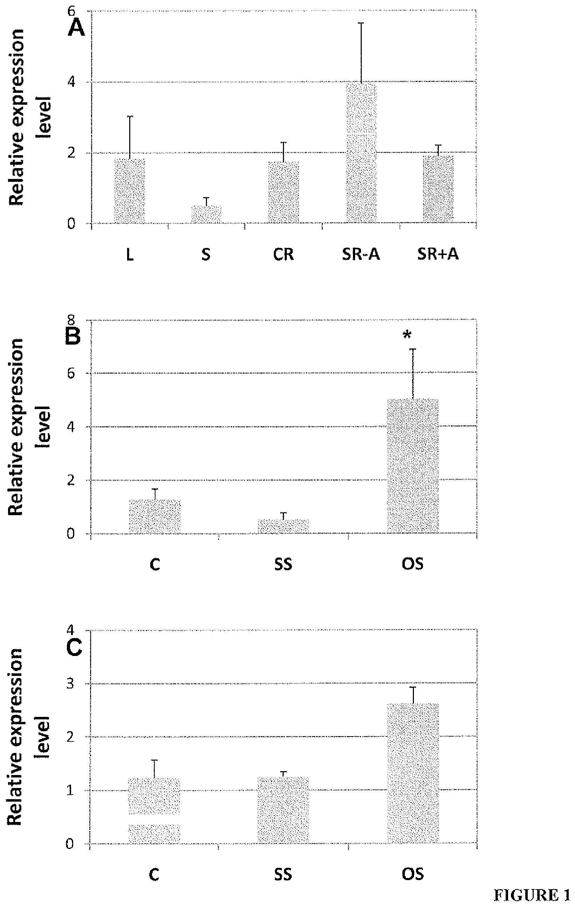

**Figure 1****:** Constitutive expression of the OsMADS26 gene. QPCR analysis of the expression profile of OsMADS26. A: OsMADS26 expression in different organs from plantlet cultivated in standard condition (MS/2). L: leaf, S: stem, CR: crown root, SR-A: seminal root without apex, SR+A: seminal root apex. B-C, expression patterns of OsMADS26 in shoot (B) and in root (C) of 7 days old rice seedlings cultivated in standard condition (C), with 150 mM NaCl (SS), 100 mM manitol (OS). Values represent the mean obtained from two independent biological repetitions, bars are standard error. *: significant difference with p=0,05.

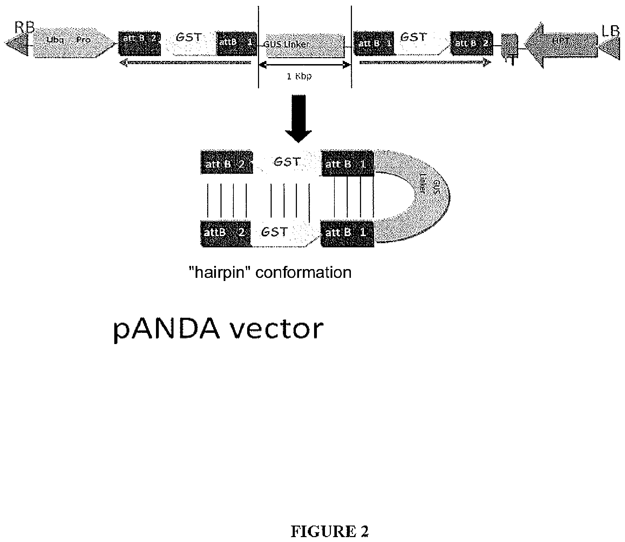

**Figure 2****:** Expression vector pANDA used for cloning OsMADS26 cDNA. The pANDA vector allows the expression under the control of the constitutive promoter of ubiquitin gene from maize of the cloned gene sequence tag (GST) in sense and antisense orientation separated by a GUS spacing sequence. The insertion of the GSTs was checked by sequencing. The obtained plasmids were named pANDA-GST1 and pANDA-GST2 (respectively for GST1 and GST2), and were transferred in an A. *tumefaciens* strain EHA105 for plant transformation.

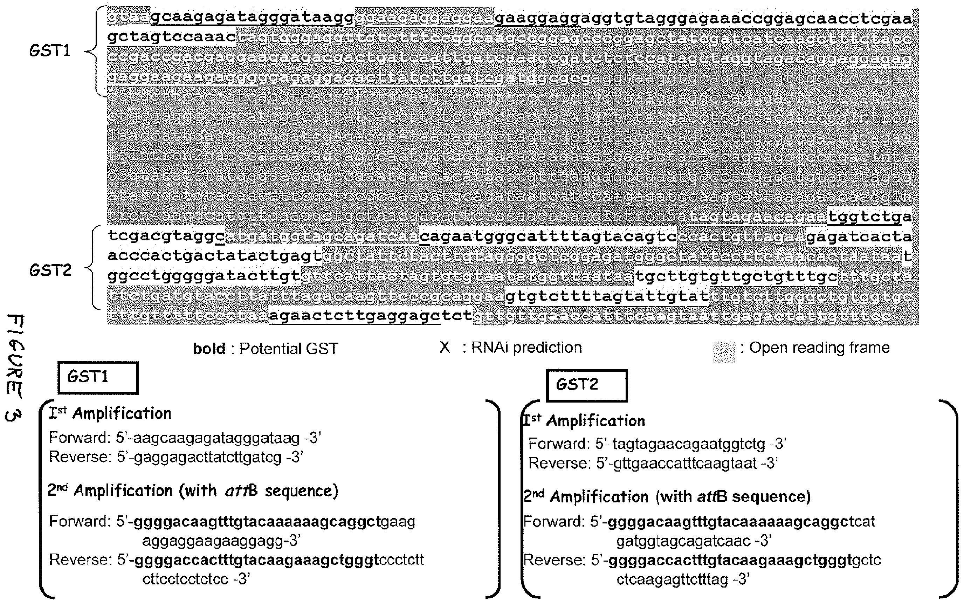

**Figure 3****:** Amplification of GST1 and GST2 sequence tags specific of MADS26-cDNA (from root of Oryza sativa) and MADS26-RNAi prediction. A PCR amplification was performed with a couple of specific primers designed in the 5' and 3' UTR of *OsMADS 26* (PC8 Forward: 5'-aagcaagagatagggataag-3', PC8 Reverse: 5'-attacttgaaatggttcaac-3'). The amplified cDNA were cloned using the pGEM-T easy cloning kit of Promega. Obtained plasmid was named pGEMT-PC8. From this plasmid further PCR reactions were done using specific primers possessing the recombination sequence for BP recombinase of the gateway cloning technology of Invitrogen in their 5' end to amplify the OsMADS26 cDNA (PC8 BP forward : 5'-ggggacaagtttgtacaaaaaagcaggctgaagaggaggaagaaggagg-3' and PC8 BP Reverse : 5'-ggggaccactttgtacaagaaagctgggtgctcctcaagagttctttag-3'), a 215 bp fragment located in the 5' UTR of OsMADS26, named GST1 (PC8 BP forward and GST1 reverse: 5'-ggggaccactttgtacaagaaagctgggtccctcttcttcctcctctcc-3') and a 321 bp fragment comprising the end of the last exon and the major part of the 3' UTR region of OsMADS26, named GST2 (GST2 forward : 5'-ggggacaagtttgtacaaaaaagcaggctcatgatggtagcagatcaac-3' and PC8 BP reverse).

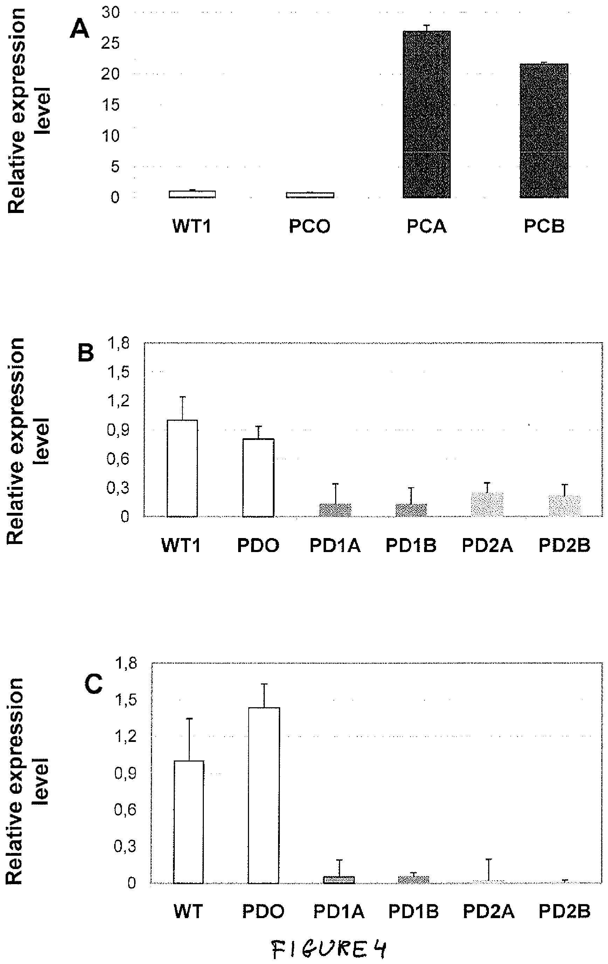

**Figure 4****:** MADS26 gene expression pattern in transgenic and RNA-interfered plants using quantitative QPCR analysis. A: *OsMADS26* expression levels in overexpressing (dark bars) and correspondant control (white bars) plants cultivated in greenhouse. **B:** *OsMADS26* expression levels in RNA interfered (grey bars) and correspondant control (white bars) plants cultivated in greenhouse. **C:** *OsMADS26* expression levels in RNA interfered (grey bars) and correspondant control (white bars) 7-d-old seedlings cultivated on MS/2 medium added with 125 mM of manitol. Values represent the mean obtained from two independent biological repetitions, bars are standard error.

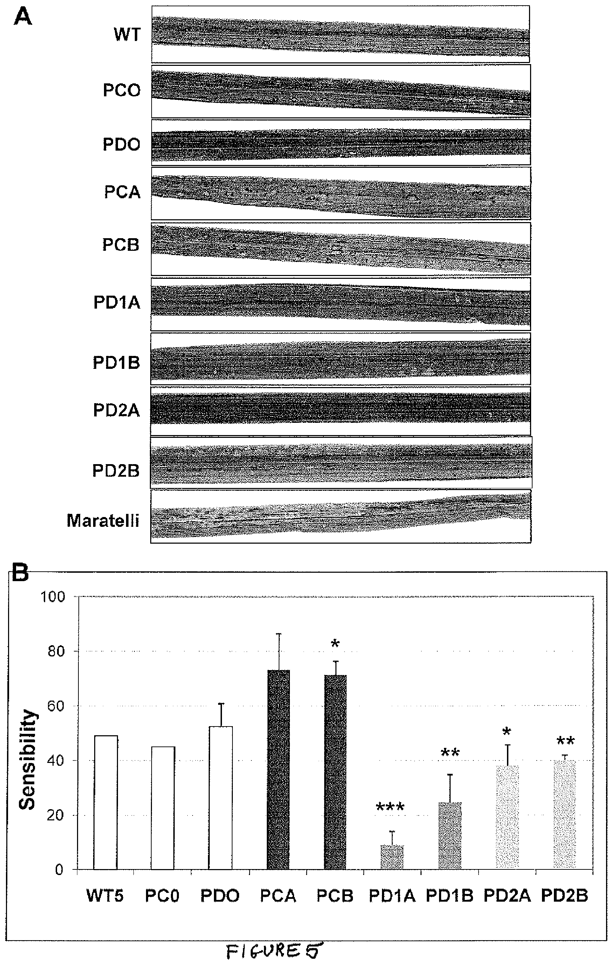

**Figure 5****:** MADS26 RNA-interfered plants are more resistant to fungal infection while plants overexpressing the MADS26 gene are less resistant to fungal infection. Resistance of OsMADS26 transgenic lines against Magnaporthe oryzae (M.oryzae). Nine independent rice lines overexpressing (PCA, PCB) (black bars) or interfered (PD1, PD2) (grey bars) OsMADS26 and corresponding control lines transformed with empty vectors (PCO, PDO) and wild-type plants (WT) (white bars) were assayed. A: Symptom severity in leaves of transgenic and control plants inoculated with the GY11 strain of M. oryzae. Photographs were taken at 3 days post inoculation. Maratelli, highly susceptible control. B: Percentage of susceptible versus total lesions observed in M. oryzae-infected leaves at 3 days after inoculation. Values represent the mean obtained from ten inoculated plants for each line, bars are corresponding standard error. Results shown are representative of the data obtained for three independent experiments. *:s ignificant difference with p<0.05; **: significant difference with p<0.01; ***: significant difference with p<0.001.

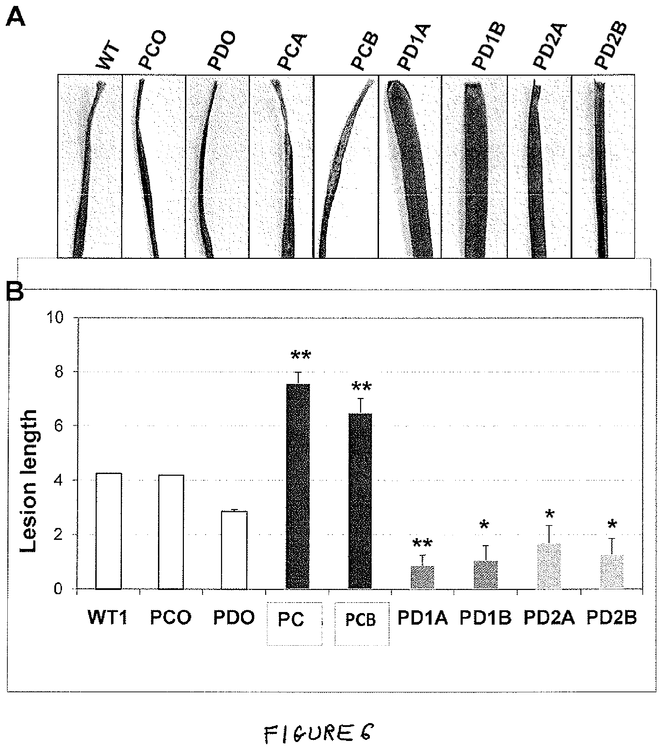

**Figure 6****:** MADS26 RNA-interfered plants are more resistant to bacterial infection while plants overexpressing the MADS26 gene are less resistant to bacterial infection. Resistance of *OsMADS26* transgenic lines against *Xanthomonas oryzae pv. Oryzae (Xoo)*. Nine independent rice lines overexpressing (PCA, PCB) (black bars) or interfered (PD1, PD2) (grey bars) *OsMADS26* and corresponding control lines transformed with empty vectors (PCO, PDO) and wild-type plants (WT) (white bars) were assayed. **A:** Symptom severity in leaves of transgenic and control plants inoculated with the POX99 strain of *Xoo.* Photographs were taken at 14 days post inoculation. **B:** Length of lesion produced in X*oo*-infected leaves at 14 dpi. Values represent the mean obtained from ten inoculated plants for each line, bars are corresponding standard error. Results shown are representative of the data obtained for two independant experiments. *: significant difference with p<0.05; **: significant difference with p<0.01.

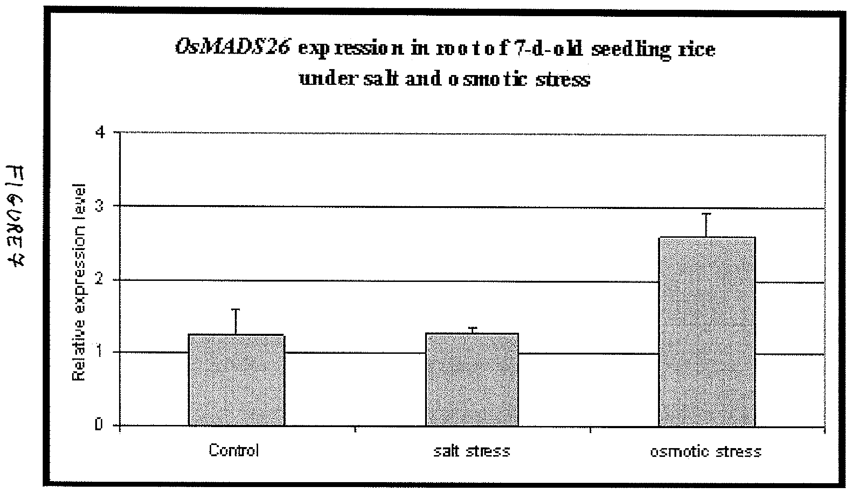

**Figure 7****:** MADS26 induction under osmotic stress. OsMADS26 gene is induced under osmotic stress.

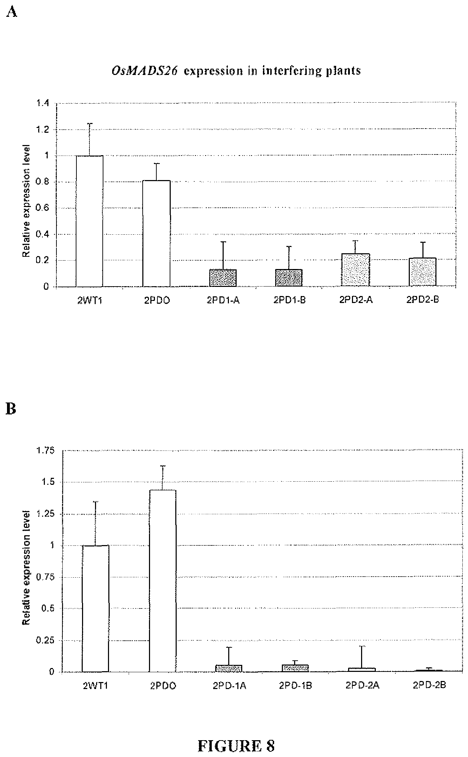

**Figure 8****:** MADS26 gene expression pattern in transgenic plants. A: OsMADS26 gene is silenced in RNAi-interfered plants (lines 2PD1-A, 2PD1-B, 2PD2-A, 2PD2-B). **B:** Under osmotic stress, MADS26 gene is still silenced.

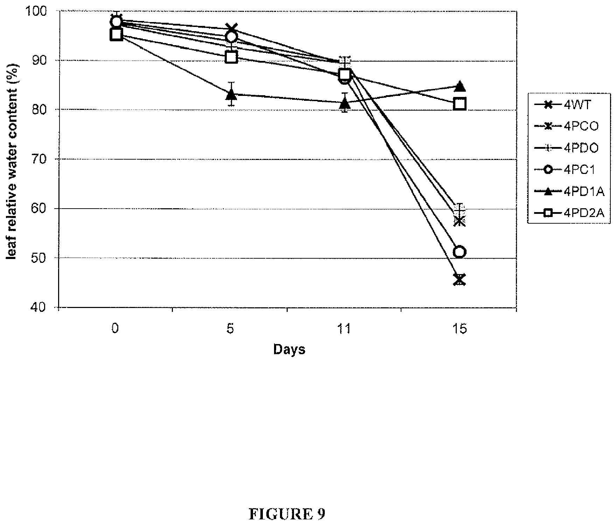

**Figure 9****:** MADS26 RNA-interfered plants are more resistant to drought stress and plants overexpressing the MADS26 gene are less resistant to drought stress. Leaf relative water content kinetics of *OsMADS26* transgenic plants during drought stress. Drought stress was applied on twenty days old plants growing in greenhouse in soil pots, by watering stopping. The values represent the mean obtained from five plants by line, bars are standard error. 4PC1, 4PC2: *OsMADS26* overexpressing plants, 4PD1A, 4PD2A: *OsMADS26* interfered plants, 4PCO, 4PDO: plants transformed with empty vectors, 4WT: untransformed plants.

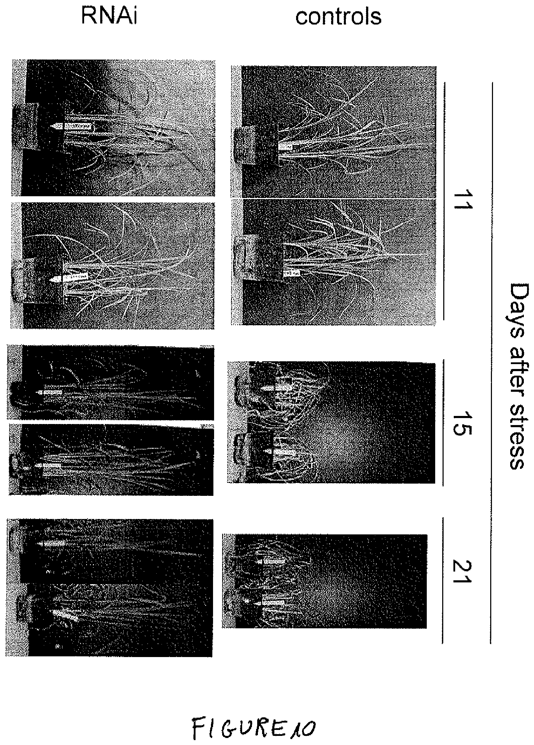

**Figure 10****:** MADS26-RNAi silenced plants are more resistant to drought stress. At the 6^{th} leaf stage, plants were not watered any more, and were kept under drought stress conditions during 21 days.

### DETAILED DESCRIPTION OF THE INVENTION

The MADS-box family of genes code for transcription factors which have a highly conserved sequence motif called MADS-box. These MADS box transcription factors have been described to control diverse developmental processes in flowering plants, ranging from root to flower and fruit development (Rounsley et al., 1995). The N-terminal part of the encoded factor seems to be the major determinant of DNA-binding specificity and the C-terminal part seems to be necessary for dimerisation.

There are several reported members of the MADS-box family of genes, including MADS26, MADS33 and MADS 14.

MADS26 gene, the rice ortholog of AGL12 in Arabidopsis thaliana, was recently proposed to be involved in senescence or maturation processes since MADS26 transcript level was increased in an age-dependent manner in leaves and roots (Lee et al., 2008). However MADS26 knock-out rice plants, which were tested under various stress conditions (such as drought, high salt, and stress mediators), showed no difference in comparison with wild-type plants.

Surprisingly, the inventors have now shown that plants with inactivated MADS26 gene are more resistant to abiotic stress such as drought stress. Moreover, the inventors have also discovered that MADS26 is a negative regulator of plant resistance to pathogens, i.e., its inhibition increases resistance. This is the first example of regulation of resistance in plants by a transcription factor of the MADS-box family. MADS-box genes thus represent novel and highly valuable targets for producing plants of interest with increased resistance to pathogens.

The present invention thus relates to methods for increasing pathogen resistance in plants based on a regulation of MADS26 gene function.

The invention also relates to plants or plant cells having an inactivated MADS-box gene function, preferably MADS26 gene function, or an ortholog thereof.

The invention also relates to constructs (e.g., nucleic acids, vectors, cells, etc) suitable for production of such plants and cells, as well as to methods for producing plant resistant regulators.

The present disclosure will be best understood by reference to the following definitions:

### Definitions

As used therein, the term "MADS-box protein" designates proteins containing a MADS-box amino acid sequence and which have a transcription factor activity. Typical MADS-box proteins bind to a DNA consensus sequence CC(A/T)₄NNGG (wherein N represents any nucleotide base), or an homogous sequence thereof. Preferred MADS-box proteins comprise the following amino acid sequence IXXXXXXXXTXXKRXXGXXKKXXEXXXL (wherein X represents any amino acid). Specific examples of a MADS-box protein include, without limitation, MADS26, MADS33 or MADS14 proteins. MADS-box have been isolated or identified in various plant species. Specific examples of MADS-box proteins include *Oryza sativa* MADS-box proteins comprising a sequence selected from SEQ ID NOs: 2, 9, or 10, *Triticum aestivum* MADS-box protein comprising a sequence of SEQ ID NO: 3, and *Hordeum vulgare* MADS-box proteins comprising a sequence selected from SEQ ID NOs: 11, 12, 13, 14 or 15. The term MADS-box proteins also encompass any variant (e.g., polymorphism) of a sequence as disclosed above, as well as orthologs of such sequences in distinct plant species.

Within the context of the present invention, the term "MADS-box gene" designates any nucleic acid that codes for a MADS-box protein as defined above. The term "MADS-box gene" includes MADS-box DNA (e.g., genomic DNA) and MADS-box RNA (e.g., mRNA). Examples of MADS-box genes include a MADS26, MADS33 or MADS14 DNA or RNA of *Oryza sativa, Triticum aestivum, Hordeum vulgare, Zea mays, Sorghum bicolor, Arabidopsis thaliana.* Specific example of a MADS-box gene comprises the nucleic acid sequence of SEQ ID NOs: 1, 4, 6 or 8.

In the most preferred embodiment, a MADS-box gene is a MADS26 gene, a MADS33 gene, a MADS 14 gene, or orthologs thereof. Within the context of the present invention, the term "ortholog" designates a related gene or protein from a distinct species, having a level of sequence identity to a reference MADS-box gene above 50% and a MADS-box gene like activity. An ortholog of a reference MADS-box gene is most preferably a gene or protein from a distinct species having a common ancestor with said reference MADS-box gene, acting as a negative regulator of plant resistance to biotic and/or abiotic stress, and having a degree of sequence identity with said reference MADS-box gene superior to 50%. Preferred orthologs of a reference MADS-box gene have least 60%, preferably at least 70 %, most preferably at least 70, 80, 90, 95% or more sequence identity to said reference sequence, e.g., to the sequence shown in SEQ ID NO: 1 (Oryza sativa). MADS-box gene orthologs can be identified using such tools as "best blast hit" searches or "best blast mutual hit" (BBMH). MADS26 orthologs have been identified by the inventors in various plants, including wheat, barley, sorghum or maize (see Table 2 and sequence listing). Specific examples of such orthologs include the nucleic acid sequence of SEQ ID NO: 4, 6 or 8, and the amino acid sequence of SEQ ID NO: 3, 5 or 7.

Further examples of MADS-box genes or proteins are listed below: Rice (Oryza sativa) GenBank:
Os12g10520.1
Os12g10520.2
Os03g54160.1
Os03g54160.2
Os07g41370.1
Os07g01820.3
Os07g01820.2
Os06g06750.1
Os07g01820.4
Os01g66290.2
Os01g66290.1
Os03g11614.1
Os03g03100.1
Os02g45770.1
Os01g52680.1

### Wheat (Triticum aestivum)

### GenBank:

CAM59056
AM502878.1
DQ512350.1
AM502870.1
DQ534490.1
DQ512331.1
AM502886.1
AM502877.1
DQ512370.1
DQ512334.1
AM502867.1
AB295661.1
AB295660.1
AB295659.1
DQ512345.1
AM502903.1
DQ534492.1
DQ512347.1
AM502868.1
DQ512351.1
AB295664.1
DQ512356.1
DQ512348.1
AM502901.1
AM502900.1

### Maize (Zea mays)

### GenBank:

ACG41656.1
ACR35354.1
NP_001148873.1

### Sorghum (Sorghum bicolor)

### GenBank:

X_002443744.1

Within the context of the present invention, the term "biotic stress" designates a stress that occurs as a result of damage done to plants by living organism, e.g. plant pathogens. The term "pathogens" designates all pathogens of plants in general such as bacteria, viruses, fungi, parasites or insects. More preferably the pathogens are fungal and/or bacterial pathogens. In a particular embodiment, fungal pathogens are cereal fungal pathogens. Examples of such pathogens include, without limitation, Magnaporthe, Puccinia, Aspergillus, Ustilago, Septoria, Erisyphe, Rhizoctonia and Fusarium species. In the most preferred embodiment, the fungal pathogen is Magnaporthe oryzae.

In another particular embodiment, bacterial pathogens are cereal bacterial pathogens. Examples of such pathogens include, without limitation, Xanthomonas, Ralstonia, Erwinia, Pectobacterium, Pantoea, Agrobacterium, Pseudomonas, Burkholderia, Acidovorax, Clavibacter, Streptomyces, Xylella, Spiroplasma and Phytoplasma species. In the most preferred embodiment, the bacterial pathogen is Xanthomonas oryzae.

Within the context of the present invention, the term "abiotic stress" designates a stress that occurs as a result of damage done to plants by non-living environmental factors such as drought, extreme cold or heat, high winds, salinity, heavy metals.

The invention is particularly suited to create cereals resistant to Magnaporthe and/or Xanthomonas and/or resistant to drought stress. Preferably, the cereal is selected from rice, wheat, barley, oat, rye, sorghum or maize. In the most preferred embodiment the resistant cereal is rice, for example Oryza sativa indica, Oryza sativa japonica.

Different embodiments of the present invention will now be further described in more details. Each embodiment so defined may be combined with any other embodiment or embodiments unless otherwise indicated. In particular, any feature indicated as being preferred or advantageous may be combined with any other feature or features indicated as being preferred or advantageous.

### MADS-box function-defective plants

As previously described, the present invention is based on the finding that MADS26 gene is a negative regulator of plant resistance to biotic and/or abiotic stress. The inventors have demonstrated that the inactivation of MADS26 gene increases plant resistance to fungal pathogens, bacterial pathogens and to drought stress.

The present disclosure thus relates to methods for increasing pathogen resistance and abiotic stress resistance in plants, based on a regulation of MADS-box transcription factor pathways.

The disclosure also relates to plants or plant cells having a defective MADS-box function.

The disclosure also relates to constructs (e.g., nucleic acids, vectors, cells, etc) suitable for production of such plants and cells, as well as to methods for producing plant resistant regulators.

According to a first embodiment, the invention relates to a plant or a plant cell comprising a defective MADS-box function. The term "MADS-box function" indicates any activity mediated by a MADS-box protein in a plant cell. The MADS-box function may be effected by the MADS-box gene expression or the MADS-box protein activity.

Within the context of this invention, the terms "defective", "inactivated" or "inactivation", in relation to MADS-box function, indicate a reduction in the level of active MADS-box protein in the cell or plant. Such a reduction is typically of about 20%, more preferably 30%, as compared to a wild-type plant. Reduction may be more substantial (e.g., above 50%, 60%, 70%, 80% or more), or complete (i.e., knock-out plants).

Inactivation of MADS-box function may be carried out by techniques known per se in the art such as, without limitation, by genetic means, enzymatic techniques, chemical methods, or combinations thereof. Inactivation may be conducted at the level of DNA, mRNA or protein, and inhibit the expression of the MADS-box gene (e.g., transcription or translation) or the activity of MADS-box protein.

Preferred inactivation methods affect expression and lead to the absence of production of a functional MADS-box protein in the cells. It should be noted that the inhibition of MADS-box function may be transient or permanent.

In a first embodiment, defective MADS-box gene is obtained by deletion, mutation, insertion and/or substitution of one or more nucleotides in one or more MADS-box gene(s). This may be performed by techniques known *per se* in the art, such as e.g., site-specific mutagenesis, ethyl methanesulfonate (EMS) mutagenesis, targeting induced local lesions in genomes (TILLING), homologous recombination, conjugation, etc.

The TILLING approach according to the invention aims to identify SNPs (single nucleotide polymorphisms) and/or insertions and/or deletions in a MADS-box gene from a mutagenized population. It can provide an allelic series of silent, missense, nonsense, and splice site mutations to examine the effect of various mutations in a gene.

Another particular approach is gene inactivation by insertion of a foreign sequence, e.g., through transposon mutagenesis using mobile genetic elements called transposons, which may be of natural or artificial origin.

According to another preferred embodiment, the defective MADS-box function is obtained by knock-out techniques.

In the most preferred embodiment, the defective MADS-box function is obtained by gene silencing using RNA interference, ribozyme or antisense technologies. Within the context of the present invention, the term "RNA interference" or "RNAi" designates any RNAi molecule (e.g. single-stranded RNA or double-stranded RNA) that can block the expression of MADS-box genes and/or facilitate mRNA degradation by hydridizing with the sequences of MADS-box mRNA.

In a particular embodiment, an inhibitory nucleic acid molecule which is used for gene silencing comprises a sequence that is complementary to a sequence common to several MADS-box genes or RNAs. Such a sequence may, in particular, encode the MAD-box motif. In a preferred embodiment, such an inhibitory nucleic acid molecule comprises a sequence that is complementary to a sequence present in a MADS26 gene and that inhibits the expression of a MADS26 gene. In a particular embodiment, such an RNAi molecule comprises a sequence that is complementary to a sequence of the MADS26 gene comprising the GST1 or GST2 sequence. In a preferred embodiment, such an RNAi molecule comprises a sequence producing a hairpin structure RNAi-GST1 or RNAi-GST2 (Figure 2; SEQ ID NO: 16 and 17). In another particular embodiment, such an inhibitory nucleic acid molecule comprises a sequence that is complementary to a sequence present in a MADS33 or MADS14 gene and that inhibits the expression of said MADS33 or MADS 14 gene.

As illustrated in the examples, MADS26 interfered plants are still viable, show no aberrant developmental phenotype, and exhibit increased resistance to plant pathogens and to drought stress.

MADS-box protein synthesis in a plant may also be reduced by mutating or silencing genes involved in the MADS-box protein biosynthesis pathway. Alternatively, MADS-box protein synthesis and/or activity may also be manipulated by (over)expressing negative regulators of MADS-box transcription factors. In another embodiment, a mutant allele of a gene involved in MADS-box protein synthesis may be (over)expressed in a plant.

MADS-box function inactivation may also be performed transiently, e.g., by applying (e.g., spraying) an exogenous agent to the plant, for example molecules that inhibit MADS-box protein activity.
Preferred inactivation is a permanent inactivation produced by destruction of one or more MADS-box genes, e.g., by deletion or by insertion of a foreign sequence of a fragment (e.g., at least 50 consecutive bp) of the gene sequence.

In a specific embodiment, more than one defective MADS-box gene(s) are obtained by knock-out techniques.

In another embodiment, defective MADS-box function is obtained at the level of the MADS-box protein. For example, the MADS-box protein may be inactivated by exposing the plant to, or by expressing in the plant cells e.g., regulatory elements interacting with MADS-box proteins or specific antibodies.

Thus, the MADS-box function in plant resistance may be controlled at the level of MADS-box gene, MADS-box mRNA or MADS-box protein.

In a variant, the disclosure relates to a plant with increased resistance to biotic and/or abiotic stress, wherein said plant comprises an inactivated MADS26, MADS33, or MADS14 gene, or an ortholog thereof. In another preferred embodiment, several MADS-box genes present in the plant are defective.

In another variant, the disclosure relates to a plant with increased resistance to biotic and/or abiotic stress, wherein said plant comprises at least one inactivated MAD-box protein, e.g. MADS26, MADS33 or MADS14 protein.

In another variant, the disclosure relates to a plant with increased resistance to biotic and/or abiotic stress, wherein said increased resistance is due to inactivation of a MAD-box transcription factor mRNA, preferably MADS26, MADS33 or MADS14 mRNA.

In another embodiment, the disclosure relates to transgenic plants or plant cells which have been engineered to be (more) resistant to biotic and/or abiotic stress by inactivation of MAD-box protein function. In a particular embodiment, the modified plant is a loss-of-function MADS26, MADS33 or MADS14 mutant plant, with increased resistance to biotic and/or abiotic stress.

The invention also relates to seeds of plants of the invention, as well as to plants, or descendents of plants grown or otherwise derived from said seeds, said plants having an increased resistance to pathogens.

The invention also relates to vegetal material of a plant of the invention, such as roots, leaves, flowers, callus, etc.

### Producing of MAD-box transcription factor defective resistant plants

The disclosure also provides a method for producing plants having increased resistance to biotic and/or abiotic stress, wherein the method comprises the following steps:
(a) inactivation of a MADS-box gene function in a plant cell;
(b) optionally, selection of plant cells of step (a) with inactivated MADS-box gene function;
(c) regeneration of plants from cells of step (a) or (b); and
(d) optionally, selection of a plant of (c) with increased resistance to and biotic and/or abiotic stress, said plant having a defective MADS-box gene function.

As indicated above, inactivation of the MADS-box gene can be done using various techniques. Genetic alteration in the MADS-box gene may also be performed by transformation using the Ti plasmid and *Agrobacterium* infection method, according to protocols known in the art. In a preferred method, inactivation is caused by RNA interference techniques or knock-out techniques.
According to another preferred embodiment, MADS-box transcription factor defective resistant plants are obtained by transforming plant cells with a recombinant vector expressing an RNAi molecule that silences MADS-box gene(s). Preferably, such a recombinant vector contains a gene sequence tag (GST) specific of nucleic acid sequence encoding a MAD-box transcription factor. In a particular embodiment, such an expression vector contains a sequence tag of SEQ ID NO: 16 (GST1) or a sequence tag of SEQ ID NO: 17 (GST2) which are both specific of MADD26-cDNA sequence. In a preferred embodiment, the recombinant expression vector is pANDA::MADS26, preferably pANDA-GST1 or pANDA-GST2. Typically, the expressed molecule adopts a hairpin conformation and stimulates generation of RNAi against the sequence tag, e.g. GST1 or GST2.

In the most preferred embodiment, resistant plants of the invention comprise a nucleic acid sequence expressing an RNAi molecule that inhibits the expression of a MADS26 gene, and exhibit an increased resistance to biotic and/or abiotic stress. Such a plant can produce RNAi molecules as described above.
The invention also relates to an isolated cDNA comprising a nucleic acid sequence selected from:
(a) a nucleic acid sequence selected from a nucleic acid sequence which encodes a MADS26 transcription factor or an ortholog thereof, or a fragment thereof;
(b) a nucleic acid sequence of SEQ ID NO: 1, 4, 6 or 8, or a fragment thereof ;
(c) a nucleic acid sequence which hybridizes to the sequence of (a) or (b) under stringent conditions, and encodes a MADS26 transcription factor or an ortholog thereof; and
(d) a mutant of a nucleic acid sequence of (a), (b) or (c).
Stringent hybridization/washing conditions are well known in the art. For example, nucleic acid hybrids that are stable after washing in 0.1x SSC, 0.1% SDS at 60°C. It is well known in the art that optimal hybridization conditions can be calculated if the sequence of the nucleic acid is known. Typically, hybridization conditions can be determined by the GC content of the nucleic acid subject to hybridization. Typically, hybridization conditions uses 4 - 6 x SSPE (20x SSPE contains Xg NaCl, Xg NaH2PO4 H2O and Xg EDTA dissolved to 1 1 and the pH adjusted to 7.4); 5-10x Denhardts solution (50x Denhardts solution contains 5g Ficoll), 5g polyvinylpyrrolidone, 5g bovine serum albumen; X sonicated salmon/herring DNA; 0.1-1.0%s sodium dodecyl sulphate; optionally 40-60% deionised formamide. Hybridization temperature will vary depending on the GC content of the nucleic acid target sequence but will typically be between 42-65 °C.
The present disclosure also relates to a recombinant vector comprising a nucleic acid molecule as described above. Such a recombinant vector may be used for transforming a cell or a plant in order to increase plant resistance to fungal pathogens, or to screen modulators of resistance. Suitable vectors can be constructed, containing appropriate regulatory sequences, including promoter sequences, terminator fragments, polyadenylation sequences, enhancer sequences, marker genes and other sequences as appropriate. Preferably the nucleic acid in the vector is under the control of, and operably linked to an appropriate promoter or other regulatory elements for transcription in a host cell such as a microbial, (e.g. bacterial), or plant cell. The vector may be a bi-functional expression vector which functions in multiple hosts. In a preferred aspect, the promoter is a constitutive or inducible promoter.

### Selecting of resistant plants

Selection of plant cells having a defective MADS-box gene can be made by techniques known per se to the skilled person (e.g., PCR, hybridization, use of a selectable marker gene, protein dosing, western blot, etc.).

Plant generation from the modified cells can be obtained using methods known per se to the skilled worker. In particular, it is possible to induce, from callus cultures or other undifferentiated cell biomasses, the formation of shoots and roots. The plantlets thus obtained can be planted out and used for cultivation. Methods for regenerating plants from cells are described, for example, by Fennell et al. (1992) Plant Cell Rep. 11: 567-570; Stoeger et al (1995) Plant Cell Rep. 14: 273-278.

The resulting plants can be bred and hybridized according to techniques known in the art. Preferably, two or more generations should be grown in order to ensure that the genotype or phenotype is stable and hereditary.

Selection of plants having an increased resistance to biotic and/or abiotic stress can be done by applying the pathogen to the plant or exposing a plant to abiotic stress factors, determining resistance and comparing to a *wt* plant.
Within the context of this invention, the term "increased resistance" to biotic and/or abiotic stress means a resistance superior to that of a control plant such as a wild type plant, to which the method of the invention has not been applied. The "increased resistance" also designates a reduced, weakened or prevented manifestation of the disease symptoms provoked by a pathogen or an abiotic stress factor. The disease symptoms preferably comprise symptoms which directly or indirectly lead to an adverse effect on the quality of the plant, the quantity of the yield, its use for feeding, sowing, growing, harvesting, etc. Such symptoms include for example infection and lesion of a plant or of a part thereof (e.g., different tissues, leaves, flowers, fruits, seeds, roots, shoots), development of pustules and spore beds on the surface of the infected tissue, maceration of the tissue, accumulation of mycotoxins, necroses of the tissue, sporulating lesions of the tissue, colored spots, etc. Preferably, according to the invention, the disease symptoms are reduced by at least 5% or 10% or 15%, more preferably by at least 20% or 30% or 40%, particularly preferably by 50% or 60%, most preferably by 70% or 80% or 90% or more, in comparison with the control plant.

The term "increased resistance" of a plant to biotic and/or abiotic stress also designates a reduced susceptibility of the plant towards infection with plant pathogens and/or towards damage of the plant caused by an abiotic stress factor, or lack of such susceptibility. The inventors have demonstrated, for the first time, a correlation between expression of a MADS-box gene and susceptibility towards infection. As shown in the experimental part, the overexpression of MADS26 gene promotes disease, whereas the MADS26-RNA interference increases resistance. The inventors have therefore proposed that the MADS-box transcription factor signaling increases susceptibility of plants to infection and favors the development of the disease due to biotic and/or abiotic factors.

Preferred plants or cells of the disclosure are MADS-box RNA interfered plants, preferably MADS26, MAD 33 or MADS 14 RNA interfered plants.

In the most preferred embodiment, the method of the invention is used to produce monocot plants having a defective MADS26 gene, with increased resistance to fungal, bacterial pathogens and/or to drought stress. Examples of such plants and their capacity to resist pathogens and drought are disclosed in the experimental section.

Further aspects and advantages of the invention are provided in the following examples, which are given for purposes of illustration and not by way of limitation.

### EXAMPLES

### 1. Materials and methods - Plant material and culture conditions

All experiments were done with *Oryza sativa japonica,* cv 'Nipponbare. For seedlings obtaining, rice seeds were dehulled and surface disinfected by immersion in 70% ethanol for 1 min, rinsed with sterile distilled water and treated with 3.84 % solution of sodium hypochlorite in 30 mn. Finally seeds were rinsed five times with sterile distilled water. Seeds were incubated in sterile distilled water in growth chamber (16 h of light per day, 500 µE m⁻² s⁻¹, 28°C/25°C day/night) for 2 days. Seeds were transferred in rectangular dishes (245 mm x 245 mm, Corning, USA, 7 seeds per dish) containing 250 ml of half Muashige and Skoog (Duchefa) standard medium (MS/2) solidified by 8 g/L of agar type II (Sigma). Theses dishes were transferred and placed vertically in growth chamber. After 7 days of culture, seedlings organs were sampled and used for RT-QPR. Saline and osmotic stresses were applied by adding in the culture medium 150 mM NaCl (Duchefa) or 100 mM manitol (Duchefa), respectively (see Figure 1). Plants were cultured in soil pots (3L, Tref, EGO 140 www.Trefgroup.com) in containment greenhouse (16-h-light/8-h-dark cycles, at 28°C to 30°C). For plant growth phenotyping, the plants belonging to the different lines were randomly arranged in the greenhouse to avoid position effect on plant growth. Twenty days after germination (DAG), plant height identified from stem base to tip of the top-most leaf on the main tiller and tiller number were measured one time per week until flowering beginning. The flowering beginning was defined as the date when the first spikelet appeared on the plant. The flowering date records the date when spikelets were observed on 50% of the tillers of the plant. After harvesting, the dry weight of the whole plant part, except the root were determined after drying the plants at 70°C for 96 h. All panicles of each plant were also weighted after dried at 37°c for 3 days. Then the percentage of seed fertility and the weight of 1000 seeds were measured on the main panicle. This experiment was repeated two times with three plants per line. Statistical analysis of data obtained in these experiments was performed using the ANOVA test with a confidence level of 5%. Specific culture conditions used for pathogen and drought resistance tests are detailed in the corresponding sections.

### 2. Plasmid construction for plant transformation

The isolation of *OsMADS26* (Os08g02070) cDNA was done by RT-PCR. Total RNA were extracted from 100mg of 7 day old seedlings grounded in liquid nitrogen using 1ml of TRIzol (Invitrogen) following the recommendation of the supplier. RNA (20 µg) was incubated with 1 unit of DNase RQ1 (Promega), 1.4 units of RNAsin (Promega) and 20mM MgCl2 in RNAse-free sterile water, for 30min at 4 °C. RNA (2µg) was denatured for 5 min at 65 °C and reverse-transcribed with 22.5 µM of oligodT(15) primer (Promega), with 10 u of AMV reverse transcriptase (Promega) for 90 min at 42°C. A PCR amplification was performed with a couple of specific primers designed in the 5' and 3' UTR of *OsMADS 26* (PC8 Forward: 5'-aagcaagagatagggataag-3', PC8 Reverse: 5'-attacttgaaatggttcaac-3'). The amplified cDNA were cloned using the pGEM-T easy cloning kit of Promega. Obtained plasmid was named pGEMT-PC8. From this plasmid further PCR reactions were done using specific primers (see Figure 3) possessing the recombination sequence for BP recombinase of the gateway cloning technology of Invitrogen in their 5' end to amplify the OsMADS26 cDNA (PC8 BP forward : 5'-ggggacaagtttgtacaaaaaagcaggctgaagaggaggaagaaggagg-3' and PC8 BP Reverse : 5'-ggggaccactttgtacaagaaagctgggtgctcctcaagagttctttag-3'), a 215 bp fragment located in the 5' UTR of OsMADS26, named GST1 (PC8 BP forward and GST1 reverse: 5'-ggggaccactttgtacaagaaagctgggtccctcttcttcctcctctcc-3') and a 321 bp fragment comprising the end of the last exon and the major part of the 3' UTR region of OsMADS26, named GST2 (GST2 forward : 5'-ggggacaagtttgtacaaaaaagcaggctcatgatggtagcagatcaac-3' and PC8 BP reverse) (see Figure 3). PCR cycling conditions were: 94 °C for 4 min (1 cycle) and 94 °C for 1 min, an annealing step at various temperatures depending on the *T*m of the primers used (typically *T*m -5 °C), for 1.5 min, and 72 °C for 1 min (35 cycles) with a 5 min final extension step at 72 °C. PCR was performed in a final volume of 25 µl with 0.25 u of *Taq* polymerase in MgCl2-free buffer (Promega), 2 mM MgCl2, 200 nM each dNTP, appropriate oligonucleotides (1µM) and cDNA (2 µl) or pGEMT-PC8 plasmid (10 ng). The BP tailed OsMADS26 cDNA was cloned with the BP recombinase in a PCAMBIA 5300 overexpression modified binary vector named PC5300.OE (see Table 1) where the ccdb gene surrounded by the BP recombination sites were cloned between the constitutive promoter of ubiquitin gene from maize and the terminator of the nopaline syntase gene from *A*. *tumefaciens*. After cloning the presence of the OsMADS26 cDNA was verified by sequencing. The plasmid named PC5300.OE-PC8 was transferred into *A. tumefaciens* strain EHA105. The BP tailed GST1 and GST2 were cloned by BP recombination in the pDON207 entry plasmid (Invitrogen) and transferred with the LR recombinase (Invitrogen) in the binary plasmid pANDA (Miki and Shimamoto, 2004).

The pANDA vector (see Figure 2) allows the expression under the control of the constitutive promoter of ubiquitin gene from maize of the cloned GST in sense and antisense orientation separated by a GUS spacing sequence. The expressed molecule adopts a hairpin conformation and stimulates the generation of siRNA against the GST sequence. The insertion of the GSTs was checked by sequencing. The obtained plasmids were named pANDA-GST1 and pANDA-GST2, and were transferred in an *A*. *tumefaciens* strain EHA105 for plant transformation.

**Table 1: List of transgenic lines obtained by the method of the invention, control lines and cloned vectors.**

| **Lines** | **Name** | **Cloned Vector** |
|---|---|---|
| Overexpressing (PC) | PC-A | pCAMBIA5300.OE |
| | PC-B | |
| RNAi (GST1) | PD1-A | pANDA |
| | PD1-B | |
| RNAi (GST2) | PD2-A | pANDA |
| | PD2-B | |
| Empty control | PCO | pCAMBIA5300.OE |
| Empty control | PDO | pANDA |
| Wildtype | WT | |

### 3. Plant transformation and selection

Transgenic plants were obtained by co-culture of seed embryo-derived callus with Agrobacterium strain EHA105 carrying the adequate binary plasmids following the procedure detailed in Sallaud et al., (2003). Monolocus and homozygotes lines were selected on the basis of the segregation of the antibiotic resistance gene carried by the TDNA. Antibiotic resistance essays were done on 5 days old seedlings incubated in Petri dishes for five days on Watman 3MM paper imbibed with 6 ml of 0.3 mg (5.69.10⁻⁴M) of hygromicin. The presence and the number of the transgenic constructions in plant genome were analyzed by Southern blot. Total genomic DNA was extracted from 200 mg grounded leaf tissue of transgenic (T0 and T1 generation) and control plants using 900 µl of mixed alkyl trimethyl ammonium bromide (MATAB) buffer (100 mM Tris-HCl, pH 8.0, 1.5 M NaCl, 20 mM EDTA, 2% (w/v) MATAB, 1% (w/v) Polyethylen glycol (PEG) 6000, 0.5% (w/v) Na₂SO₂) and incubated at 72°C for 1 h. The mixture was then cooled to room temperature for 10 mn, and 900 µl of chloroform: isoamyl alcohol (24:1, v/v) was added. After mixing and sedimentation at 6000g for 10 mn, the aqueous phase was transferred in a new 1.5 ml Eppendorf tube and 20U of RNase A were added, the mix was incubated at 37°C for 30 mn. RNAse A was eliminated by a new treatment with 900 µl of Chloroform: isoamyl alcohol (24:1, v/v) and the genomic DNA was finally precipitated after addition of 0.8 volume of isopropanol to the aqueous phase. To evaluate the number of T-DNA insertions in the genome of transgenic plants, 5µg of genomic DNA were cleaved overnight at 37°C with 20 units of *SacI* or *Kpn1* (Biolabs) which cut in only one position the TDNA derived from PC5300.OE or pANDA vectors, respectively. DNA fragments were separated by electrophresis in 0.8% agarose gel with TAE buffer (0.04 M Tris-acetate, 0.001 M EDTA). After incubation for 15x mn in 1L of 0.25N HCL then in 1L of 0.4N NaOH for 30 mn, DNA was transferred by capillarity in alkaline conditions (0.4N NaOH) onto a Hybond N+ membrane (Amersham Biosciences). The membranes were prehybridized for 4h at 65°C in a buffer containing 50 mM Tris-HCl pH 8, 10 mM EDTA pH 8, 5X SSC, 0.2% SDS (w/v) (Eurobio, France), 1X Denhardt's solution (Denhart 50X, Sigma, ref. 2532) and 50µg of fragmented salmon sperm DNA. Hybridization was performed overnight at 65°C in a buffer containing 50 mM Tris-HCl pH 8, 10 mM EDTA pH 8, 5X SSC, 0.2% SDS (w/v) (Eurobio, France), 1X Denhardt's solution (Denhart 50X, Sigma, ref. 2532), 40µg DNA of fragmented salmon sperm DNA and 10% Dextran sulphate (w/v). To check for TDNA copy numbers 80 ng of a 550 bp fragment of the hygromicin resistance gene *hph,* labelled with [α-³²P] with the random priming kit (Amersham^{™}, UK) was denaturated 10 mn at 95°C and added to the hybridization mixture. After hybridization, the membranes were washed at 65°C, for 15 mn in 80 ml of buffer S1 containing 2X SSC, 0.5% SDS (Eurobio, France) (v/v), for 30 mn in 50 ml of buffer S2 containing 0.5X SSC and 0.1% SDS (v/v) and finally for 30 mn in 50 ml of buffer S3 containing 0.1X SSC and 0.1% SDS (v/v). The membranes were put in contact with a radiosensible screen (Amersham Bioscience, "Storage Phosphor Screen unmounted 35x43", ref. 63-0034-80) for 2-3 days. Revelation was performed with a phosphoimageur scanner (Storm 820, Amersham). In order to check for the complete integration of the constructions allowing *OsMADS26* constitutive expression or expression of the hairpin molecules designed with specific OsMADS26 GSTs, plant genomic DNA were cleaved with *Kpn1* and *BamH1* or *Sac1* and *Kpn1* respectively. Southern blot were done using [α-³²P] labelled specific probes of ORF8 or GST1 or GST2 depending of the construction (see Figure 3). The expression of OsMADS26 in selected transgenic lines was analyzed by RT-QPCR.

### 4. Real-time quantitative reverse trauscriptase polymerase chain reactions (RT-qPCR) analysis

Plant material was collected, immediately frozen in liquid nitrogen, and stored at -80°C. Tissues were ground in liquid nitrogen. Total RNA were extracted from 100 mg grounded tissues with 1ml of TRIzol (Invitrogen) following the recommendation of the supplier. Total RNA were quantified according to their absorbance at 260 nm with a nanoquant Tecan-Spectrophotometer. Five µg of RNA were treated to remove residual genomic for 30 mn at 37°C DNA with 5U of DNAse RQ1 (Promega) and 1 µl of RQ1 RNAse-Free DNAse 10X reaction buffer in a final volume of 10 µl. Then, 1 µl of RQ1 DNAse Stop Solution was added to terminate the reaction and the mix was incubated at 65°C for 10 mn to inactivate the DNAse. The first strand cDNA synthesis was done in 20 µl of final volume using the kit Superscripts III (Invitrogen) following the manufacturer's instructions. The presence of genomic DNA in sample was checked by a PCR reaction using 1µl of cDNA as template and primers: Act-F (5'-ggcttctctcagcaccttccagc-3'), Act-R (5'-cgatatctggagcaaccaaccaca-3') designed in two exons surrounding an intron of the actin encoding gene (Os01g73310.1). The PCR was done in a thermocycler Techne (TC-512) as follows: 95°C for 3 min; 30 to 35 cycles of 95°C for 30 sec, 60°C for 1 min, and 72°C for 1 min; with a final extension at 72°C for 7 min. The PCR was done with 0,5 U of Taq polymerase in a final volume of 50 µl of the corresponding buffer (Biolab) and 2mM MgCl₂ (Biolab), 0,08 mM of dNTP (Fermentas) and 0,02 µM of each specific primers. Ten microliters from the 50-µL PCR product was separated on a 1% (w/v) agarose gel in 1X TAE buffer and visualized under UV after staining with (6 drops/L) ethidium bromide. For RT-qPCR analysis of gene expression pattern specific forward (F) and reverse (R) primers were designed to amplify a fragment of 200-400 bp in 3' untranslated zone (3'-UTR) of each studied gene using the Vector NTI (version 10.1) software with default parameters. The RT-qPCR was performed with LighCycler 480 system (Roche) using the SYBR green master mix (Roche) containing optimized buffer, dNTP and Taq DNA polymerase, and manufactured as described in the user manual. The reaction was carried out in 96-well optical reaction plates (Roche). The reaction mix contained 7.5 µl SYBR Green QPCR Master Mix (Roche), 250 nm of each primer (F and R), and 3µl of 10 fold diluted cDNA template. All reactions were heated to 95°C for 5min, followed by 45 cycles of 95°C for 10s and 60°C for 30s. Melt curve analysis and gel electrophoresis of the PCR products were used to confirm the absence of non-specific amplification products. Transcripts from an EP gene (Expressed Protein, Os06g11070.1) were also detected and used as an endogenous control to normalize expression of the other genes. EP was chosen as the housekeeping gene because its expression appeared to be the most stable in different tissues and physiological conditions (Canada et al, 2007). Relative expression level were calculated by subtracting the Ct (threshold cycle) values for EP from those of the target gene (to give ΔCₜ), then ΔΔCₜ and calculating 2^{-ΔΔCt} (Giulietti et al. 2001). Reactions were performed in triplicate to provide technical replicates and all experiments were replicated at least once with similar results.

Results: the inventors have confirmed that MADS26 expression in OsMADS26 mRNA-interfered plants PD1A, PD1B, PD2A et PD2B was silenced (Figure 4B and D) while the MADS26 expression level in PCA and PCB transgenic plants over-expressing the OsMADS26 is at least 20-fold more important than the MADS26 expression in control plants (Figure 4A).

### 5. Resistance assay against Magnaporthe oryzae

In addition to the studied transgenic lines, *O*. *sativa* japonica cv Maratelli was used as a susceptible control. Plants were sown in trays of 40x29x7 cm filled with compost of Neuhaus S pH 4-4.5 and Pozzolana (70 liters Neuhaus S mixed with 2 shovels of Pozzolana). Ten seeds of each line were sown in rows in a tray containing 12 lines each. Plants were grown until the 4-5 leaf stage a greenhouse with a thermoperiod of 26/21°C (day/night), a 12-h photoperiod under a light intensity of 400-600 W/m². Watering was done every day and once a week nutritive solution composed of 1.76 g/L of Algospeed (Laboratoire Algochimie, Chateau-Renault, France) and 0.125 g/L of Ferge (FERVEG, La Rochelle, France) was supplied. The GUY 11 isolate (CIRAD collection, Montpellier, France) of *M. oryzae* was used for inoculation. This isolate is compatible with O. Sativa cv Nipponbare and generate partial susceptible symptoms. The fungus was cultured in Petri dishes containing 20ml of medium composed of 20gl/l rice seed flour prepared grounding paddy rice at machine (Commerciel Blendor American) for 3 mn, 2.5g/l yeast extract (Roth-2363.3), 1.5% agar (VWP, 20768.292) supplemented after autoclaving with 500 000 units /L of sterile penicillin G (Sigma P3032-10MU). Fungus culture was carried out in a growth chamber with a 12-h photoperiod and a constant temperature of 25°C for 7 days. After 7 days, conidia were harvested from plates by flooding the plate with 10 ml of sterile distilled water and filtering through two layers of gauze to remove mycelium fragment from the suspension. The concentration in conidia of the suspension was adjusted to 50000 conidia ml⁻¹ and supplemented with 0.5% (w:v) of gelatin (Merck). Inoculations were performed on 4-5 leaf stage plantlets by spraying 30 ml of the conidia suspensions on each tray. Inoculated plantlets were incubated for 16h in a controlled climatic chamber at 25°C, 95% relative humidity and transferred back to the greenhouse. After 3 to 7 days, lesions on rice leaves were categorized in resistant or susceptible categories and counted. The data presented are representative of data obtained for three independent repetitions of the experimentation.

Results: the inventors have demonstrated in Figure 5 that OsMADS26 mRNA interfered plants PD1A, PD1B, PD2A et PD2B are more resistant to fungal pathogens while PCA and PCB plants over-expressing the OsMADS26 gene are more susceptible to fungal diseases.

### 6. Resistance assay against Xanthomonas oryzae pv. Oryzae (Xoo)

Resistance assays against *Xanthomonas oryzae* pv. *Oryzae (Xoo)* were carried out on 2 month-old rice plants grown in the same conditions as described above for *M. oryzae* resistance assays. After 2 months, the plants were transferred from greenhouse to a culture chamber providing 12h light at 28°C (5 tubes fluorescent) and 12h obscurity (0 tubes fluorescents) at 21°C circadian cycles. In order to evaluate expression of genes identified as markers of defence in the different studied lines in the absence of pathogen, one month before infection, the youngest and the before youngest fully expended leaf were collected pooling 3 plantlets in the same line. This sample was used for QPCR analysis with specific primers of defence genes. *The* Xoo strain PXO99, a representative strain of Philippines race 6 (Song et al. 1995) was grown on PSA medium (10 gl⁻¹ peptone, 10 g/L sucrose, 1 g/L glutamic acid, 16 g/L bacto-agar, pH 7.0) for 3 days at 27°C. Bacterial blight inoculation was performed using the leaf-clipping methode described by Kauffman et al. (1973). The bacterial cells of *Xoo* were suspended in 50 ml sterile water to obtain an optical density of 0.5 measured at 600nm (OD600). The bacterial cell suspension was applied to the two youngest fully expanded leaves on the main tiller of 2 months old rice plants by cutting the leaf 5-6 cm from the tip using a pair of scissors dipped in the Xoo solution. Lesion length (LL) was measured 14 days post-inoculation (dpi) according to the criteria described previously (Amante-Bordeos et al. 1992). The data presented are representative of data obtained for two independent repetitions of the experimentation. After symptom measurement, infected leaves were also collected in liquid nitrogen and used for RNA extraction and QPCR analysis to measure the expression level of different defence genes.

Results: the inventors have demonstrated in Figure 6 that OsMADS26 mRNA interfered plants PD1A, PD1B, PD2A et PD2B are more resistant to bacterial pathogens while PCA and PCB plants over-expressing the OsMADS26 gene are more susceptible to bacterial diseases. Indeed, PCA and PCB plants have much more lesions than PD1A, PD1B, PD2A et PD2B plants.

### 7. Resistance assay against water stress

Plants were germinated in a one-half-strength MS liquid medium in a growth chamber for 7 d and transplanted into soil and grown in the green house at the same conditions described above. Each pot was filled with the same amount of soils (Tref, EGO 140), planted with 5 seedlings and watered with the same volume of water. After one month, plants were subjected to 18 days of withholding water followed by 15 days of watering. Drought tolerance was evaluated by determining the percentage of plants that survived or continued to grow after the period of recovery. Fv/Fm values of plants were measured each day after withholding watering with a pulse modulated fluorometer (Handy PEA, EUROSEP Instruments) as previously described (Jang et al. 2003; Oh et al. 2005). This experiment was done on 20 plants per line and repeated three times. Statistical analysis of the data obtained in these experiments was performed using the R software at a 5% confidence level. During water stress, the relative water content (RWC), of leaves was measured according to Barrs and Weatherly, 1962. A mid-leaf section of about 1 x 7 cm was cut with scissors from the top of the most expanded leaf of five plants. The other leaves were also harvested, frozen in liquid nitrogen and stocked at -80°C for RNA extraction and RT-qPCR analysis of the expression of stress related genes. For RWC measurement, each leaf section was pre-weighed airtight to obtain leaf sample weight (W). After that, the sample was immediately hydrated to full turgidity. The basal part of the leaf was placed to the bottom of a caped 50 ml Stardet tube containing 15 ml of de-ionized water and incubated at room temperature. After 4 h, the leaf was removed and dried quickly and lightly with filter paper and immediately weighed to obtain fully turgid weight (TW). Sample were then dried at 80°C for 24 h and weighed to determine dry weight (DW). The RWC was calculated as following: RWC (%) = [(W-DW) / (TW-DW)] x 100. Basis on the results of this calculation, the samples stocked at -80°C of two plants were taken out. RNA extraction and RT-qPCR were performed from two plants of each line that had the same RWC, as described earlier with specific primers of genes identified as drought and high salinity stresses markers in rice: rab21, a rice dehydrin (accession number AK109096), salT (salt-stress-induced protein, accession number AF001395), and dip1 (dehydration-stress inducible protein 1, accession number AY587109) genes (Claes et al. 1990; Oh et al. 2005; Rabbani et al. 2003).

Results: the inventors have discovered that OsMADS26 gene is induced under osmotic stress (Figure 7) and that the OsMADS26 expression profile is different in various plant organs (Figure 1). The inventors have also demonstrated that OsMADS26 gene is silenced in RNAi-interfered plants (lines 2PD1-A, 2PD1-B, 2PD2-A, 2PD2-B) (Figure 8A) and that under osmotic stress, the MADS26 gene is still silenced (Figure 8B). Finally, in Figures 9 and 10, the inventors have demonstrated that MADS26 RNA-interfered plants are more resistant to drought stress and plants overexpressing the MADS26 gene are less resistant to drought stress.

### 8. MADS26 orthologs

Furthermore, the inventors have carried out Tblastn searches with the MADS26 protein from rice and have identified by blastp search several putative orthologs in wheat, sorghum and maize. To see if homology uncovers phylogenetic relationship and possibly functional homology, the inventors have tested whether the cereal homologs were in turn the best blast hit (Best Blast Mutual Hit=BBMH) on rice.

**Table 2: Orthologs of MADS26**

| **Species** | **SEQ ID NO MADS26 best homolog** | **Best homolog Accession (1)** | **% Amino acid identity** |
|---|---|---|---|
| wheat | SEQ ID NO: 3 | CAM59056 | 65% |
| sorghum | SEQ ID NO: 5 | XP_002443744.1 | 66% |
| maize | SEQ ID NO: 7 | ABW84393 | 85% |

| | | | |
|---|---|---|---|
| (1) The MADS26 protein SEQ ID NO: 2 was searched against wheat, sorghum and maize sequences using blastp in the ncbi sequence database. | | | |

### Conclusions

Altogether, the expression data and the phenotypical data indicate that the MADS26 gene is a negative regulator of resistance to Magnaporthe oryzae, to Xanthomonas oryzae and to drought stress. This is the first example ever found of a plant transcription factor of the MADS-box family negatively regulating biotic and abiotic stress response.

### Sequence listing

**SEQ ID NO: 1**
**SEQ ID NO: 2**
**SEQ ID NO: 3**
   Putative TaMADS26 The amino acid sequence of SEQ IS NO: 4 derives from
**SEQ ID NO: 4**
**SEQ ID NO: 5**
   Putative sorghum MADS26 The amino acid sequence of SEQ ID NO: 6 derives from
**SEQ ID NO: 6**
**SEQ ID NO: 7**
   Putative Zea mays MADS26 The amino acid sequence of SEQ ID NO: 8 derives from
**SEQ ID NO: 8**
**SEQ ID NO: 9**
**SEQ ID NO: 10**
**SEQ ID NO: 11**
   Putative HvMADS26
**SEQ ID NO: 12**
   Putative HvMADS26
**SEQ ID NO: 13**
   Putative HvMADS26
**SEQ ID NO: 14**
   Putative HvMADS26
**SEQ ID NO: 15**
   Putative HvMADS26
**SEQ ID NO: 16**
   GST1 (215bp) specific of OsMADS26 (see Figure 3)
**SEQ ID NO: 17**
   GST2 (321bp) specific of OsMADS26 (see Figure 3)

### REFERENCES

Alvarez-Buylla, E.R., Liljegren, S.J., Pelaz, S., Gold, S.E., Burgeff, C., Ditta, G.S., Vergara-Silva, F., and Yanofsky, M.F. (2000). MADS-box gene evolution beyond flowers: expression in pollen, endosperm, guard cells, roots and trichomes. Plant J 24, 457-466.
Arora, R., Agarwal, P., Ray, S., Singh, A.K., Singh, V.P., Tyagi, A.K., and Kapoor, S. (2007). MADS-box gene family in rice: genome-wide identification, organization and expression profiling during reproductive development and stress. BMC Genomics 8, 242.
Fang, S.C., and Fernandez, D.E. (2002). Effect of regulated overexpression of the MADS domain factor AGL15 on flower senescence and fruit maturation. Plant Physiol 130, 78-89**.**
Fernandez, D.E., Heck, G.R., Perry, S.E., Patterson, S.E., Bleecker, A.B., and Fang, S.C. (2000). The embryo MADS domain factor AGL15 acts postembryonically. Inhibition of perianth senescence and abscission via constitutive expression. Plant Cell 12, 183-198.
Khush, G. (2005) What it will take to Feed 5.0 Billion Rice consumers in 2030. Plant Molecular Biology, 59(1): 1-6.
Khush, GS. and Jena, KK., (2009) Current status and future prospects for research on blast resistance in rice (Oryza sativa L.) In GL Wang (ed.) and B Valent (ed.). Advances in Genetics, Genomics and Control of Rice Blast Disease, p.1-10, Springer.
Lee, S., Woo, Y.M., Ryu, S.I., Shin, Y.D., Kim, W.T., Park, K.Y., Lee, I.J., and An, G. (2008). Further characterization of a rice AGL12 group MADS-box gene, OsMADS26. Plant Physiol 147, 156-168.
Liljegren, S.J., Ditta, G.S., Eshed, Y., Savidge, B., Bowman, J.L., and Yanofsky, M.F. (2000). SHATTERPROOF MADS-box genes control seed dispersal in Arabidopsis. Nature 404, 766-770.
Mao, L., Begum, D., Chuang, H.W., Budiman, M.A., Szymkowiak, E.J., Irish, E.E., and Wing, R.A. (2000). JOINTLESS is a MADS-box gene controlling tomato flower abscission zone development. Nature 406, 910-913.
Messenguy, F., and Dubois, E. (2003). Role of MADS box proteins and their cofactors in combinatorial control of gene expression and cell development. Gene 316, 1-21.
Montiel, G., Breton, C., Thiersault, M., Burlat, V., Jay-Allemand, C., and Gantet, P. (2007). Transcription factor Agamous-like 12 from Arabidopsis promotes tissue-like organization and alkaloid biosynthesis in Catharanthus roseus suspension cells. Metab Eng 9, 125-132.
Parenicova, L., de Folter, S., Kieffer, M., Horner, D.S., Favalli, C., Busscher, J., Cook, H.E., Ingram, R.M., Kater, M.M., Davies, B., Angenent, G.C., and Colombo, L. (2003). Molecular and phylogenetic analyses of the complete MADS-box transcription factor family in Arabidopsis: new openings to the MADS world. Plant Cell 15, 1538-1551.
Shore, P., and Sharrocks, A.D. (1995). The MADS-box family of transcription factors. Eur J Biochem 229, 1-13.
Tapia-Lopez, R., Garcia-Ponce, B., Dubrovsky, J.G., Garay-Arroyo, A., Perez-Ruiz, R.V., Kim, S.H., Acevedo, F., Pelaz, S., and Alvarez-Buylla, E.R. (2008). An AGAMOUS-related MADS-box gene, XAL1 (AGL12), regulates root meristem cell proliferation and flowering transition in Arabidopsis. Plant Physiol 146, 1182-1192.
Theissen, G., Becker, A., Di Rosa, A., Kanno, A., Kim, J.T., Munster, T., Winter, K.U., and Saedler, H. (2000). A short history of MADS-box genes in plants. Plant Mol Biol 42, 115-149.
Vrebalov, J., Ruezinsky, D., Padmanabhan, V., White, R., Medrano, D., Drake, R., Schuch, W., and Giovannoni, J. (2002). A MADS-box gene necessary for fruit ripening at the tomato ripening-inhibitor (rin) locus. Science 296, 343-346.

## Claims

1. A plant belonging to the monocots having a defective MADS26 function as a result of gene silencing induced by RNA interference, said plant exhibiting an increased resistance to biotic and/or abiotic stress.

2. The plant of claim 1, wherein said plant is a cereal selected from rice, wheat, barley, oat, rye, sorghum or maize.

3. A seed of the plant of claim 2.

4. The plant of any one of claims 1 to 3, wherein said resistance to biotic stress is a resistance to fungal and/or bacterial pathogens.

5. The plant of claim 4, wherein said fungal pathogens are selected from *Magnaporthe, Puccinia, Ustilago, Septoria, Erisyphe, Rhizoctonia* and *Fusarium* species, preferably the pathogen is *Magnaporthe oryzae*.

6. The plant of claim 4, wherein said bacterial pathogens are selected from *Xanthomonas, Ralstonia, Erwinia, Pectobacterium, Pantoea, Agrobacterium, Pseudomonas, Burkholderia, Acidovorax, Clavibacter, Streptomyces, Xylella, Spiroplasma* and *Phytoplasma* species, preferably the pathogen is *Xanthomonas oryzae*.

7. The plant of claim 1, wherein said resistance to abiotic stress is a resistance to drought stress.

8. A method for increasing stress resistance to fungal and/or bacterial pathogens or to drought stress in a monocot plant, wherein the method comprises the following steps:
(a) inactivation of MADS26 gene function in a plant cell or a seed;
(b) optionally, selection of plant cells of step (a) with inactivated MADS26 gene function;
(c) regeneration of plants from cells of step (a) or (b); and
(d) optionally, selection of a plant of (c) with increased resistance to fungal and/or bacterial pathogens or to drought stress, said plant having a defective MADS26 gene function.

9. The method according to claim 8, wherein said MADS26 gene function is inactivated by deletion, insertion and/or substitution of one or more nucleotides, site-specific mutagenesis, ethyl methanesulfonate (EMS) mutagenesis, targeting induced local lesions in genomes (TILLING), knock-out techniques, or by gene silencing induced by RNA interference.

10. The method of any one of claims 8 or 9, comprising:
(a) inactivation of MADS26 gene function in seeds by mutagenesis;
(b) generation of plantlets from the seeds of step (a); and
(c) selection of a plantlet of (b) having a defective MADS26 gene function.

11. The method according to anyone of claims 8 to 10, wherein the plant is a monocot from the *Poaceae* family, preferably selected from rice, wheat, barley, oat, rye, sorghum or maize.

12. An RNAi molecule that binds to MAD26 mRNA sequence and that inhibits the expression of the MAD26 gene, said RNAi being optionally complementary to a sequence comprising the sequence of SEQ ID NO: 16 (GST1) or SEQ ID NO: 17 (GST2).

13. Use of an RNAi molecule according to claim 12, for increasing resistance of plants or plant cells belonging to the monocots to biotic or abiotic stress.

14. A method for producing a monocot plant having increased resistance to fungal and/or bacterial pathogens or to drought stress, wherein the method comprises the following steps:
(a) inactivation of MADS26 gene function in a plant cell or a seed as a result of gene silencing induced by RNA interference;
(b) optionally, selection of plant cells of step (a) with inactivated MADS26 gene function;
(c) regeneration of plants from cells of step (a) or (b); and
(d) optionally, selection of a plant of (c) with increased resistance to fungal and/or bacterial pathogens or to drought stress, said plant having a defective MADS26 gene function as a result of gene silencing induced by RNA interference.

15. A plant belonging to the monocots transformed with a vector comprising a nucleic acid sequence expressing an RNAi molecule that binds to MAD26 mRNA sequence, and is optionally complementary to a sequence comprising the sequence of SEQ ID NO: 16 (GST1) or SEQ ID NO: 17 (GST2), and that inhibits the expression of MADS26 gene.

## Patentansprüche

1. Eine zu den Einkeimblättrigen gehörende Pflanze mit einer defekten MADS26-Funktion, wobei die defekte MADS26-Funktion das Ergebnis einer durch RNA-Interferenz induzierten Genabschaltung ist, wobei die Pflanze eine erhöhte Resistenz gegen biotischen und/oder abiotischen Stress aufweist.

2. Die Pflanze nach Anspruch 1, wobei die Pflanze ein Getreide ausgewählt aus Reis, Weizen, Gerste, Hafer, Roggen, Hirse oder Mais ist.

3. Ein Samen der Pflanze nach Anspruch 2.

4. Die Pflanze nach einem der Ansprüche 1 bis 3, wobei die Resistenz gegen biotischen Stress eine Resistenz gegen pilzartige und/oder bakterielle Pathogene ist.

5. Die Pflanze nach Anspruch 4, wobei die pilzartigen Pathogene ausgewählt sind aus *Magnaporthe, Puccinia, Ustilago, Septoria, Erisyphe, Rhizoctonia* und *Fusarium* Species, vorzugsweise das Pathogen *Magnaporthe oryzae* ist.

6. Die Pflanze nach Anspruch 4, wobei die bakteriellen Pathogene ausgewählt sind aus *Xanthomonas, Ralstonia, Erwinia, Pectobacterium, Pantoea, Agrobacterium Pseudomonas, Burkholderia, Acidovorax, Clavibacter, Streptomyces, Xylella, Spiroplasma* und *Phytoplasma* Species, vorzugsweise das Pathogen *Xanthomonas oryzae* ist.

7. Die Pflanze nach Anspruch 1, wobei die Resistenz gegen abiotischen Stress eine Resistenz gegen Trockenstress ist.

8. Ein Verfahren zur Erhöhung der Stressresistenz gegen pilzartige und/oder bakterielle Pathogene oder gegen Trockenstress in einer einkeimblättrigen Pflanze, wobei das Verfahren die folgenden Schritte umfasst:
(a) Inaktivieren der MADS26-Funktion in einer Pflanzenzellen oder einem Samen;
(b) gegebenenfalls Selektionieren von Pflanzenzellen aus Schritt (a) mit inaktivierter MADS26-Genfunktion;
(c) Regenerieren von Pflanzenzellen aus Schritt (a) oder (b); und
(d) gegebenenfalls Selektionieren einer Pflanze aus (c) mit erhöhter Resistenz gegen pilzartige und/oder bakterielle Pathogene oder gegen Trockenstress, wobei die Pflanze eine defekte MADS26-Genfuntion hat.

9. Das Verfahren nach Anspruch 8, wobei die MADS26-Funktion inaktiviert ist durch Deletion, Insertion und/oder Substitution einer oder mehrerer Nukleotide, ortsgerichtete Mutagenese, Ethylmethansulfonat(EMS)-Mutagenese, zielgerichtet induzierte lokale Läsionen in Genomen (TILLING), Knock-out-Techniken oder durch RNA-Interferenz induzierter Genabschaltung.

10. Das Verfahren nach einem der Ansprüche 8 oder 9, umfassend:
(a) Inaktivieren der MADS26-Funktion in Samen durch Mutagenese;
(b) Generieren von Jungpflanzen aus den Samen aus Schritt (a); und
(c) Selektionieren einer Jungpflanze aus (b) mit einer defekten MADS26-Genfuntion.

11. Das Verfahren nach einem der Ansprüche 8 bis 10, wobei die Pflanze eine Einkeimblättrige aus der *Poaceae* Familie ist, vorzugsweise ausgewählt aus Reis, Weizen, Gerste, Hafer, Roggen, Hirse oder Mais ist.

12. Ein RNAi-Molekül, das an die MADS26 mRNA-Sequenz bindet und die Expression des MADS26-Gens inhibiert, wobei das RNAi-Molekül gegebenenfalls komplementär zu einer Sequenz ist, umfassend die Sequenz nach SEQ ID NO: 16 (GST1) oder SEQ ID NO: 17 (GST2).

13. Verwendung eines RNAi-Moleküls nach Anspruch 12 zur Erhöhung der Resistenz gegen biotischen oder abiotischen Stress von zu den Einkeimblättrigen gehörenden Pflanzen oder Pflanzenzellen.

14. Ein Verfahren zur Herstellung einer einkeimblättrigen Pflanze mit erhöhter Resistenz gegen pilzartige und/oder bakterielle Pathogene oder gegen Trockenstress, wobei das Verfahren die folgenden Schritte umfasst:
(a) Inaktivieren der MADS26-Funktion in einer Pflanzenzellen oder einem Samen als Ergebnis einer durch RNA-Interferenz induzierten Genabschaltung;
(b) gegebenenfalls Selektionieren von Pflanzenzellen aus Schritt (a) mit inaktivierter MADS26-Genfunktion;
(c) Regenerieren von Pflanzenzellen aus Schritt (a) oder (b); und
(d) gegebenenfalls Selektionieren einer Pflanze aus (c) mit erhöhter Resistenz gegen pilzartige und/oder bakterielle Pathogene oder gegen Trockenstress, wobei die Pflanze eine defekte MADS26-Genfuntion als Ergebnis einer durch RNA-Interferenz induzierten Genabschaltung hat.

15. Eine zu den Einkeimblättrigen gehörende Pflanze transformiert mit einem Vektor, umfassend eine Nukleinsäuresequenz, die ein RNAi-Molekül exprimiert, welches an die MADS26 mRNA-Sequenz bindet, und die gegebenenfalls komplementär zu einer Sequenz ist, umfassend die Sequenz nach SEQ ID NO: 16 (GST1) oder SEQ ID NO: 17 (GST2), und die die Expression des MADS26-Gens inhibiert.

## Revendications

1. Plante appartenant aux monocotylédones ayant une fonction MADS26 défectueuse suite à une extinction de gène induite par ARN interférence, ladite plante présentant une résistance accrue aux stress biotiques et/ou abiotiques.

2. Plante selon la revendication 1, dans laquelle ladite plante est une céréale sélectionnée parmi le riz, le blé, l'orge, l'avoine, le seigle, le sorgho ou le maïs.

3. Graine de la plante selon la revendication 2.

4. Plante selon l'une quelconque des revendications 1 à 3, dans laquelle ladite résistance à un stress biotique est une résistance aux pathogènes fongiques et/ou bactériens.

5. Plante selon la revendication 4, dans laquelle les pathogènes fongiques sont sélectionnés parmi les espèces *Magnaporthe, Puccinia, Ustilago, Septoria, Erisyphe, Rhizoctonia* et *Fusarium,* préférentiellement le pathogène est *Magnaporthe oryzae.*

6. Plante selon la revendication 4, dans laquelle les pathogènes bactériens sont sélectionnés parmi les espèces *Xanthomonas, Ralstonia, Erwinia, Pectobacterium, Pantoea, Agrobacterium, Pseudomonas, Burkholderia, Acidovorax, Clavibacter, Streptomyces, Xylella, Spiroplasma* et *Phytoplasma,* préférentiellement le pathogène est *Xanthomonas oryzae.*

7. Plante selon la revendication 1, dans laquelle la résistance à un stress abiotique est une résistance à la sécheresse.

8. Méthode pour augmenter la résistance au stress dû à des pathogènes fongiques et/ou bactériens ou à la sécheresse dans une plante monocotylédone, dans laquelle la méthode comprend les étapes suivantes :
(a) inactivation de la fonction du gène MADS26 dans une cellule de plante ou une graine ;
(b) optionnellement, sélection de cellules de plante de l'étape (a) avec une fonction du gène MADS26 inactivée ;
(c) régénération de plantes à partir de cellules de l'étape (a) ou (b) ; et
(d) optionnellement, sélection d'une plante de (c) avec une résistance accrue aux pathogènes fongiques et/ou bactériens ou à la sécheresse, ladite plante ayant une fonction du gène MADS26 défectueuse.

9. Méthode selon la revendication 8, dans laquelle la fonction du gène MADS26 est inactivée par délétion, insertion et/ou substitution d'un ou plusieurs nucléotides, par mutagénèse site-spécifique, mutagénèse éthyle méthanosulfonate (EMS), lésions du génome ciblées induites localement (TELLING), technique de knock-out, ou par extinction de gène induite par ARN interférence.

10. Méthode selon l'une quelconque des revendications 8 ou 9, comprenant :
(a) inactivation de la fonction du gène MADS26 dans des graines par mutagénèse ;
(b) génération de plants à partir des graines de l'étape (a) ;
(c) sélection d'un plant de (b) ayant une fonction du gène MADS26 défectueuse.

11. Méthode selon l'une quelconque des revendications 8 à 10, dans laquelle la plante est une monocotylédone de la famille de *Poaceae,* préférentiellement sélectionnée parmi le riz, le blé, l'orge, l'avoine, le seigle, le sorgho ou le maïs.

12. Molécule d'ARNi qui se lie à la séquence d'ARNm de MADS26 et qui inhibe l'expression du gène MADS26, ledit ARNi étant optionnellement complémentaire d'une séquence comprenant la séquence SEQ NO :16 (GST1) ou SEQ NO :17 (GST2).

13. Utilisation d'une molécule d'ARNi selon la revendication 12, pour augmenter la résistance de plantes ou de cellules de plantes appartenant aux monocotylédones à un stress biotique ou abiotique.

14. Méthode de production d'une plante monocotylédone ayant une résistance accrue aux pathogènes fongiques et/ou bactériens ou à la sécheresse, dans laquelle la méthode comprend les étapes suivantes :
(a) inactivation de la fonction du gène MADS26 dans une cellule de plante ou une graine suite à l'extinction de gène induite par ARN interférence ;
(b) optionnellement, sélection de cellules de plante de l'étape (a) avec une fonction du gène MADS26 inactivée ;
(c) régénération de plantes à partir de cellules de l'étape (a) ou (b) ; et
(d) optionnellement, sélection d'une plante de (c) avec une résistance accrue aux pathogènes fongiques et/ou bactériens ou à la sécheresse, ladite plante ayant une fonction du gène MADS26 défectueuse suite à l'extinction de gène induite par ARN interférence.

15. Plante appartenant aux monocotylédones transformée avec un vecteur comprenant une séquence d'acide nucléique exprimant une molécule d'ARNi qui se lie à la séquence d'ARNm de MADS26, et est optionnellement complémentaire d'une séquence comprenant la séquence SEQ NO :16 (GST1) ou SEQ NO :17 (GST2), et qui inhibe l'expression du gène MADS26.


### SEQUENCE LISTING

<110> Institut National de la Recherche Agronomique et al.
<120> STRESS RESISTANT PLANTS AND THEIR PRODUCTION
<130> B1091
<150> US61/410,074
   <151> 2010-11-04
<160> 17
<170> PatentIn version 3.3
<210> 1
   <211> 669
   <212> DNA
   <213> oryza sativa
<400> 1
   atggcgcgag gcaaggtgca gctccgtcgc atcgagaacc cggttcaccg tcaggtcacc     60
   ttctgcaagc gccgtgccgg cctgctgaag aaggccaggg agctctccat cctctgcgag    120
   gccgacatcg gcatcatcat cttctccgcc cacggcaagc tctacgacct cgccaccacc    180
   ggaaccatgg aggagctgat cgagaggtac aagagtgcta gtggcgaaca ggccaacgcc    240
   tgcggcgacc agagaatgga cccaaaacag gaggcaatgg tgctcaaaca agaaatcaat    300
   ctactgcaga agggcctgag gtacatctat gggaacaggg caaatgaaca catgactgtt    360
   gaagagctga atgccctaga gaggtactta gagatatgga tgtacaacat tcgctccgca    420
   aagatgcaga taatgatcca agagatccaa gcactaaaga gcaaggaagg catgttgaaa    480
   gctgctaacg aaattctcca agaaaagata gtagaacaga atggtctgat cgacgtaggc    540
   atgatggtag cagatcaaca gaatgggcat tttagtacag tcccactgtt agaagagatc    600
   actaacccac tgactatact gagtggctat tctacttgta ggggctcgga gatgggctat    660
   tccttctaa                                                            669
<210> 2
   <211> 280
   <212> PRT
   <213> oryza sativa
<400> 2
   Met Ala Arg Gly Lys Val Gln Leu Arg Arg Ile Glu Asn Pro Val His
   1               5                   10                  15
   Arg Gln Val Thr Phe Cys Lys Arg Arg Ala Gly Leu Leu Lys Lys Ala
               20                  25                  30
   Arg Glu Leu Ser Ile Leu Cys Glu Ala Asp Ile Gly Ile Ile Ile Phe
           35                  40                  45
   Ser Ala His Gly Lys Leu Tyr Asp Leu Ala Thr Thr Gly Thr Met Glu
       50                  55                  60
   Glu Leu Ile Glu Arg Tyr Lys Ser Ala Ser Gly Glu Gln Ala Asn Ala
   65                  70                  75                  80
   Cys Gly Asp Gln Arg Met Asp Pro Lys Gln Glu Ala Met Val Leu Lys
                   85                  90                  95
   Gln Glu Ile Asn Leu Leu Gln Lys Gly Leu Arg Tyr Ile Tyr Gly Asn
               100                 105                 110
   Arg Ala Asn Glu His Met Gly Thr Met Glu Glu Leu Ile Glu Arg Tyr
           115                 120                 125
   Lys Ser Ala Ser Gly Glu Gln Ala Asn Ala Cys Gly Asp Gln Arg Met
       130                 135                 140
   Asp Pro Lys Gln Glu Ala Met Val Leu Lys Gln Glu Ile Asn Leu Leu
   145                 150                 155                 160
   Gln Lys Gly Leu Arg Tyr Ile Tyr Gly Asn Arg Ala Asn Glu His Met
                   165                 170                 175
   Thr Val Glu Glu Leu Asn Ala Leu Glu Arg Tyr Leu Glu Ile Trp Met
               180                 185                 190
   Tyr Asn Ile Arg Ser Ala Lys Met Gln Ile Met Ile Gln Glu Ile Gln
           195                 200                 205
   Ala Leu Lys Ser Lys Glu Gly Met Leu Lys Ala Ala Asn Glu Ile Leu
       210                 215                 220
   Gln Glu Lys Ile Val Glu Gln Asn Gly Leu Ile Asp Val Gly Met Met
   225                 230                 235                 240
   Val Ala Asp Gln Gln Asn Gly His Phe Ser Thr Val Pro Leu Leu Glu
                   245                 250                 255
   Glu Ile Thr Asn Pro Leu Thr Ile Leu Ser Gly Tyr Ser Thr Cys Arg
               260                 265                 270
   Gly Ser Glu Met Gly Tyr Ser Phe
           275                 280
<210> 3
   <211> 224
   <212> PRT
   <213> triticum aestivum
<400> 3
   Met Ala Arg Gly Lys Val Gln Leu Arg Arg Ile Glu Asn Pro Val His
   1               5                   10                  15
   Arg Gln Val Thr Phe Cys Lys Arg Arg Ala Gly Leu Leu Lys Lys Ala
               20                  25                  30
   Arg Glu Leu Ser Val Leu Cys Asp Ala Asp Ile Gly Ile Ile Ile Phe
           35                  40                  45
   Ser Ala His Gly Lys Leu Tyr Asp Leu Ala Thr Thr Gly Thr Met Asp
       50                  55                  60
   Gly Leu Ile Glu Arg Tyr Lys Ser Ala Ser Gly Glu Gly Met Thr Gly
   65                  70                  75                  80
   Asp Gly Cys Gly Asp Gln Arg Val Asp Pro Lys Gln Glu Ala Met Val
                   85                  90                  95
   Leu Lys Gln Glu Ile Asp Leu Leu Gln Lys Gly Leu Arg Tyr Ile Tyr
               100                 105                 110
   Gly Asn Arg Ala Asn Glu His Met Asn Val Asp Glu Leu Asn Ala Leu
           115                 120                 125
   Glu Arg Tyr Leu Glu Ile Trp Met Phe Asn Ile Arg Ser Ala Lys Met
       130                 135                 140
   Gln Ile Met Ile Gln Glu Ile Gln Ala Leu Lys Ser Lys Glu Gly Met
   145                 150                 155                 160
   Leu Lys Ala Ala Asn Glu Ile Leu Gln Glu Lys Ile Val Glu Gln His
                   165                 170                 175
   Gly Leu Ile Asp Val Gly Met Thr Ile Ala Asp Gln Gln Asn Gly His
               180                 185                 190
   Phe Ser Thr Val Pro Met Leu Glu Glu Ile Thr Asn Pro Leu Thr Ile
           195                 200                 205
   Leu Ser Gly Tyr Ser Thr Cys Arg Gly Ser Glu Met Gly Tyr Ser Phe
       210                 215                 220
<210> 4
   <211> 675
   <212> DNA
   <213> triticum aestivum
<400> 4
   atggcgagag gcaaggtcca gctccggcgc atcgagaacc ccgtccaccg gcaggtcacc     60
   ttctgcaagc gccgcgcagg gctcctcaag aaggccaggg agctctctgt cctctgcgac    120
   gccgacatcg gcatcatcat cttctccgca cacggcaagc tctacgacct cgccaccacc    180
   ggaaccatgg atgggctgat cgagaggtac aagagtgcca gtggagaagg catgaccggc    240
   gacggctgcg gcgaccagag agtggaccca aagcaggagg caatggtgct gaaacaagaa    300
   atagaccttc tgcagaaggg actgaggtac atttatggaa acagggcaaa tgagcacatg    360
   aatgttgacg agctgaatgc cctggagagg tacttggaga tatggatgtt caacatccgc    420
   tccgcaaaga tgcagataat gattcaagag atccaggcac tgaagagcaa ggagggcatg    480
   ttgaaagctg ccaacgaaat tctccaggaa aagatagtag aacagcatgg actgatcgac    540
   gtaggcatga ctatagcaga tcagcagaat gggcatttta gtacagtccc aatgttagag    600
   gagatcacta acccactgac tatactgagt ggctattcta cttgtagggg ctcagagatg    660
   ggctattcct tctga                                                     675
<210> 5
   <211> 222
   <212> PRT
   <213> sorghum bicolor
<400> 5
   Met Ala Arg Gly Lys Val Gln Leu Arg Arg Ile Glu Asn Pro Val His
   1               5                   10                  15
   Arg Gln Val Thr Phe Cys Lys Arg Arg Ala Gly Leu Leu Lys Lys Ala
               20                  25                  30
   Arg Glu Leu Ser Val Leu Cys Asp Ala His Ile Gly Ile Ile Ile Phe
           35                  40                  45
   Ser Ala His Gly Lys Leu Tyr Asp Leu Ala Thr Thr Gly Thr Met Glu
       50                  55                  60
   Glu Leu Ile Asp Arg Tyr Lys Thr Ala Ser Gly Glu Ala Ala Asp Gly
   65                  70                  75                  80
   Ser Gly Asp Asn Arg Met Asp Pro Lys Gln Glu Thr Met Val Leu Gln
                   85                  90                  95
   Gln Glu Ile Asn Leu Leu Gln Lys Gly Leu Arg Tyr Ile Tyr Gly Asn
               100                 105                 110
   Arg Ala Asn Glu His Met Asn Val Asp Glu Leu Asn Ala Leu Glu Arg
           115                 120                 125
   Tyr Leu Glu Ile Trp Met Tyr Asn Ile Arg Ser Ala Lys Met Gln Ile
       130                 135                 140
   Met Ile Gln Glu Ile Gln Ala Leu Lys Ser Lys Glu Gly Met Leu Lys
   145                 150                 155                 160
   Ala Ala Asn Glu Ile Leu Arg Glu Lys Ile Val Glu Gln Ser Ser Leu
                   165                 170                 175
   Leu Asp Val Gly Met Val Val Ala Asp Gln Gln Asn Gly His Phe Ser
               180                 185                 190
   Thr Val Pro Leu Ile Glu Glu Ile Thr Asn Pro Leu Thr Ile Leu Ser
           195                 200                 205
   Gly Tyr Ser Asn Cys Arg Gly Ser Glu Met Gly Tyr Ser Phe
       210                 215                 220
<210> 6
   <211> 669
   <212> DNA
   <213> sorghum bicolor
<400> 6
   atggcgcggg gcaaagtgca gctgcggcgc atcgagaacc cggtgcaccg gcaggtgacc 60
   ttctgcaagc gccgcgcggg gctgctcaag aaggcacggg agctctccgt cctctgcgac 120
   gcccacatcg gcatcatcat cttctccgcg cacggcaagc tctacgacct cgccaccacc 180
   gggaccatgg aagagctgat cgacaggtac aagactgcca gcggagaagc tgccgacggc 240
   tccggcgaca acagaatgga tccaaaacaa gaaaccatgg tgctgcaaca ggaaatcaat 300
   ctgctccaga aaggactcag gtacatctac gggaacaggg caaatgaaca catgaatgtt 360
   gacgaactga atgcccttga gaggtacttg gagatatgga tgtacaacat ccgctctgca 420
   aagatgcaga taatgattca agagatacaa gcactaaaaa gcaaggaagg catgttgaaa 480
   gctgctaacg aaattctccg ggaaaagata gtagaacaga gtagtttgct tgatgtaggc 540
   atggtggtag cggatcaaca gaatgggcat tttagtacag tcccactgat agaagagatc 600
   actaacccac tgactatact gagtggatat tctaactgta ggggctcaga gatgggctat 660
   tccttctaa 669
<210> 7
   <211> 245
   <212> PRT
   <213> zea mays
<400> 7
   Met Gly Arg Gly Lys Val Gln Leu Lys Arg Ile Glu Asn Lys Ile Asn
   1               5                   10                  15
   Arg Gln Val Thr Phe Ser Lys Arg Arg Ser Gly Leu Leu Lys Lys Ala
               20                  25                  30
   His Glu Ile Ser Val Leu Cys Asp Ala Glu Val Ala Leu Ile Ile Phe
           35                  40                  45
   Ser Thr Lys Gly Lys Leu Tyr Glu Tyr Ser Thr Asp Ser Cys Met Asp
       50                  55                  60
   Lys Ile Leu Asp Arg Tyr Glu Arg Tyr Ser Tyr Ala Glu Lys Val Leu
   65                  70                  75                  80
   Ile Ser Val Glu Ser Glu Thr Gln Gly Asn Trp Cys His Glu Tyr Arg
                   85                  90                  95
   Lys Leu Lys Ala Lys Val Glu Thr Ile Gln Lys Cys Gln Lys His Leu
               100                 105                 110
   Met Gly Glu Asp Leu Glu Thr Leu Asn Leu Lys Glu Leu Gln Gln Leu
           115                 120                 125
   Glu Gln Gln Leu Glu Ser Ser Leu Lys His Ile Arg Thr Arg Lys Ser
       130                 135                 140
   Gln Leu Met Leu Glu Ser Ile Ser Glu Leu Gln Arg Lys Glu Lys Ser
   145                 150                 155                 160
   Leu Gln Glu Glu Asn Lys Val Leu Gln Lys Glu Leu Ala Glu Lys Gln
                   165                 170                 175
   Lys Ala Gln Arg Lys Gln Val Gln Trp Gly Gln Thr Gln Gln Gln Thr
               180                 185                 190
   Ser Ser Ser Ser Ser Cys Phe Met Ile Arg Glu Ala Ala Pro Thr Thr
           195                 200                 205
   Asn Ile Ser Ile Phe Pro Val Ala Ala Gly Gly Arg Leu Val Glu Gly
       210                 215                 220
   Ala Ala Ala Gln Pro Gln Ala Arg Val Gly Leu Pro Pro Trp Met Leu
   225                 230                 235                 240
   Ser His Leu Ser Ser
                   245
<210> 8
   <211> 738
   <212> DNA
   <213> zea mays
<400> 8
   atggggcgcg gtaaggtgca gctgaagcgg atcgagaaca agatcaaccg ccaggtgacc     60
   ttctccaagc gccgctcggg gctgctcaag aaggcgcacg agatctccgt gctctgcgac    120
   gccgaggtcg cgctcatcat cttctccacc aaagggaagc tctacgagta ttccaccgat    180
   tcatgtatgg acaaaattct tgaccggtac gagcgctact cctatgcaga aaaggttctt    240
   atttcagtag aatctgaaac tcagggcaat tggtgccacg agtatagaaa actaaaggcg    300
   aaggtcgaga caatacaaaa atgtcaaaag cacctcatgg gagaggatct tgaaacgttg    360
   aatctcaaag agcttcagca actagagcag cagctggaga gttcactgaa acatatcaga    420
   accaggaaga gccagcttat gctcgagtca atttcggagc tccaacggaa ggagaagtcg    480
   ctgcaggagg agaacaaggt tctgcagaag gagctcgcgg agaagcagaa agcccagcgg    540
   aagcaagtgc aatggggcca aacccaacag cagaccagtt cgtcttcctc gtgcttcatg    600
   ataagggaag ctgccccaac aacaaatatc agcatttttc ctgtggcagc aggcgggagg    660
   ttggtggaag gtgcagcagc gcagccacag gctcgcgttg gactaccacc atggatgctt    720
   agccacctga gcagctga                                                  738
<210> 9
   <211> 202
   <212> PRT
   <213> oryza sativa
<400> 9
   Met Val Arg Gly Lys Val Gln Met Arg Arg Ile Glu Asn Pro Val His
   1               5                   10                  15
   Arg Gln Val Thr Phe Cys Lys Arg Arg Gly Gly Leu Leu Lys Lys Ala
               20                  25                  30
   Arg Glu Leu Ser Val Leu Cys Asp Ala Asp Val Gly Val Ile Ile Phe
           35                  40                  45
   Ser Ser Gln Gly Lys Leu His Glu Leu Ala Thr Asn Gly Asn Met His
       50                  55                  60
   Asn Leu Val Glu Arg Tyr Gln Ser Asn Val Ala Gly Gly Gln Met Glu
   65 70 75 80
   Pro Gly Ala Leu Gln Arg Gln Gln Val Ala Glu Gln Gly Ile Phe Leu
   85 90 95
   Leu Arg Glu Glu Ile Asp Leu Leu Gln Arg Gly Leu Arg Ser Thr Tyr
   100 105 110
   Gly Gly Gly Ala Gly Glu Met Thr Leu Asp Lys Leu His Ala Leu Glu
   115 120 125
   Lys Gly Leu Glu Leu Trp Ile Tyr Gln Ile Arg Thr Thr Lys Met Gln
   130 135 140
   Met Met Gln Gln Glu Ile Gln Phe Leu Arg Asn Lys Glu Gly Ile Leu
   145 150 155 160
   Lys Glu Ala Asn Glu Met Leu Gln Glu Lys Val Lys Glu Gln Gln Lys
   165 170 175
   Leu Tyr Met Ser Leu Leu Asp Leu His Ser Gln Gln Pro Thr Gln Pro
   180 185 190
   Met Thr Tyr Gly Asn Arg Phe Phe Ser Ile
   195 200
<210> 10
   <211> 253
   <212> PRT
   <213> oryza sativa
<400> 10
   Met Gly Arg Gly Lys Val Gln Leu Lys Arg Ile Glu Asn Lys Ile Asn
   1               5                   10                  15
   Arg Gln Val Thr Phe Ser Lys Arg Arg Ser Gly Leu Leu Lys Lys Ala
               20                  25                  30
   Asn Glu Ile Ser Val Leu Cys Asp Ala Glu Val Ala Leu Ile Ile Phe
           35                  40                  45
   Ser Thr Lys Gly Lys Leu Tyr Glu Tyr Ala Thr Asp Ser Cys Met Asp
       50                  55                  60
   Lys Ile Leu Glu Arg Tyr Glu Arg Tyr Ser Tyr Ala Glu Lys Val Leu
   65                  70                  75                  80
   Ile Ser Ala Glu Ser Asp Thr Gln Gly Asn Trp Cys His Glu Tyr Arg
                   85                  90                  95
   Lys Leu Lys Ala Lys Val Glu Thr Ile Gln Lys Cys Gln Lys His Leu
               100                 105                 110
   Met Gly Glu Asp Leu Glu Ser Leu Asn Leu Lys Glu Leu Gln Gln Leu
           115                 120                 125
   Glu Gln Gln Leu Glu Asn Ser Leu Lys His Ile Arg Ser Arg Lys Ser
       130                 135                 140
   Gln Leu Met Leu Glu Ser Ile Asn Glu Leu Gln Arg Lys Glu Lys Ser
   145                 150                 155                 160
   Leu Gln Glu Glu Asn Lys Val Leu Gln Lys Glu Asn Pro Cys Ser Phe
                   165                 170                 175
   Leu Gln Leu Val Glu Lys Gln Lys Val Gln Lys Gln Gln Val Gln Trp
               180                 185                 190
   Asp Gln Thr Gln Pro Gln Thr Ser Ser Ser Ser Ser Ser Phe Met Met
           195                 200                 205
   Arg Glu Ala Leu Pro Thr Thr Asn Ile Ser Asn Tyr Pro Ala Ala Ala
       210                 215                 220
   Gly Glu Arg Ile Glu Asp Val Ala Ala Gly Gln Pro Gln His Val Arg
   225                 230                 235                 240
   Ile Gly Leu Pro Pro Trp Met Leu Ser His Ile Asn Gly
                   245                 250
<210> 11
   <211> 265
   <212> PRT
   <213> hordeum vulgare
<400> 11
   Met Gly Arg Gly Pro Val Gln Leu Arg Arg Ile Glu Asn Lys Ile Asn
   1               5                   10                  15
   Arg Gln Val Thr Phe Ser Lys Arg Arg Ser Gly Leu Leu Lys Lys Ala
               20                  25                  30
   His Glu Ile Ser Val Leu Cys Asp Ala Glu Val Ala Leu Ile Val Phe
           35                  40                  45
   Ser Thr Lys Gly Lys Leu Tyr Glu Tyr Ser Ser Gln Asp Ser Ser Met
       50                  55                  60
   Asp Val Ile Leu Glu Arg Tyr Gln Arg Tyr Ser Phe Glu Glu Arg Ala
   65                  70                  75                  80
   Val Leu Asp Pro Ser Thr Gly Asp Gln Ala Asn Trp Gly Asp Glu Tyr
                   85                  90                  95
   Gly Ser Leu Lys Ile Lys Leu Asp Ala Leu Gln Lys Ser Gln Arg Gln
               100                 105                 110
   Leu Leu Gly Glu Gln Leu Asp Pro Leu Thr Thr Lys Glu Leu Gln Gln
           115                 120                 125
   Leu Glu Gln Gln Leu Asp Ser Ser Leu Lys His Ile Arg Ser Arg Lys
       130                 135                 140
   Asn Gln Leu Leu Phe Glu Ser Ile Ser Glu Leu Gln Lys Lys Glu Lys
   145                 150                 155                 160
   Ser Leu Lys Asp Gln Asn Gly Val Leu Gln Lys His Leu Val Glu Thr
                   165                 170                 175
   Glu Lys Glu Lys Asn Asn Val Leu Ser Asn Ile His His Arg Glu Gln
               180                 185                 190
   Leu Asn Glu Ala Thr Asn Ile His His Gln Glu Gln Leu Ser Gly Ala
           195                 200                 205
   Thr Thr Ser Ser Pro Ser Pro Thr Pro Pro Thr Ala Gln Asp Ser Met
       210                 215                 220
   Ala Pro Pro Asn Ile Gly Pro Tyr Gln Ser Arg Gly Gly Gly Asp Pro
   225                 230                 235                 240
   Glu Pro Gln Pro Ser Pro Ala Gln Ala Asn Asn Ser Asn Leu Pro Pro
                   245                 250                 255
   Trp Met Leu Arg Thr Ile Gly Asn Arg
               260                 265
<210> 12
   <211> 246
   <212> PRT
   <213> hordeum vulgare
<400> 12
   Met Gly Arg Gly Arg Val Glu Leu Lys Arg Ile Glu Asn Lys Ile Asn
   1               5                   10                  15
   Arg Gln Val Thr Phe Ala Lys Arg Arg Asn Gly Leu Leu Lys Lys Ala
               20                  25                  30
   Tyr Glu Leu Ser Val Leu Cys Asp Ala Glu Val Ala Leu Ile Val Phe
           35                  40                  45
   Ser Asn Arg Gly Lys Leu Tyr Glu Phe Cys Ser Thr Gln Ser Met Thr
       50                  55                  60
   Lys Thr Leu Asp Lys Tyr Gln Lys Cys Ser Tyr Ala Gly Pro Glu Thr
   65                  70                  75                  80
   Thr Val Gln Asn Arg Glu Asn Glu Gln Leu Lys Asn Ser Arg Asn Glu
                   85                  90                  95
   Tyr Leu Lys Leu Lys Thr Arg Val Asp Asn Leu Gln Arg Thr Gln Arg
               100                 105                 110
   Asn Leu Leu Gly Glu Asp Leu Asp Ser Leu Gly Ile Lys Glu Leu Glu
           115                 120                 125
   Ser Leu Glu Lys Gln Leu Asp Ser Ser Leu Lys His Ile Arg Thr Thr
       130                 135                 140
   Arg Thr Gln His Met Val Asp Gln Leu Thr Glu Leu Gln Arg Arg Glu
   145                 150                 155                 160
   Gln Met Phe Ser Glu Ala Asn Lys Cys Leu Arg Ile Lys Leu Glu Glu
                   165                 170                 175
   Ser Asn Gln Val His Gly Gln Gln Leu Trp Glu His Asn Asn Asn Val
               180                 185                 190
   Leu Ser Tyr Glu Arg Gln Pro Glu Val Gln Pro Gln Met His Gly Gly
           195                 200                 205
   Asn Gly Phe Phe His Pro Leu Asp Ala Ala Gly Glu Pro Thr Leu His
       210                 215                 220
   Ile Gly Tyr Pro Pro Glu Ser Leu Asn Ser Ser Cys Met Thr Thr Phe
   225                 230                 235                 240
   Met Pro Pro Trp Leu Pro
                   245
<210> 13
   <211> 244
   <212> PRT
   <213> hordeum vulgare
<400> 13
   Met Gly Arg Gly Lys Val Gln Leu Lys Arg Ile Glu Asn Lys Ile Asn
   1               5                   10                  15
   Arg Gln Val Thr Phe Ser Lys Arg Arg Ser Gly Leu Leu Lys Lys Ala
               20                  25                  30
   His Glu Ile Ser Val Leu Cys Asp Ala Glu Val Gly Leu Ile Ile Phe
           35                  40                  45
   Ser Thr Lys Gly Lys Leu Tyr Glu Phe Ser Thr Glu Ser Cys Met Asp
       50                  55                  60
   Lys Ile Leu Glu Arg Tyr Glu Arg Tyr Ser Tyr Ala Glu Lys Val Leu
   65                  70                  75                  80
   Val Ser Ser Glu Ser Glu Ile Gln Gly Asn Trp Cys His Glu Tyr Arg
                   85                  90                  95
   Lys Leu Lys Ala Lys Val Glu Thr Ile Gln Lys Cys Gln Lys His Leu
               100                 105                 110
   Met Gly Glu Asp Leu Glu Ser Leu Asn Leu Lys Glu Leu Gln Gln Leu
           115                 120                 125
   Glu Gln Gln Leu Glu Ser Ser Leu Lys His Ile Arg Ala Arg Lys Asn
       130                 135                 140
   Gln Leu Met His Glu Ser Ile Ser Glu Leu Gln Lys Lys Glu Arg Ser
   145                 150                 155                 160
   Leu Gln Glu Glu Asn Lys Val Leu Gln Lys Glu Leu Val Glu Lys Gln
                   165                 170                 175
   Lys Ala Gln Ala Ala Gln Gln Asp Gln Thr Gln Pro Gln Thr Ser Ser
               180                 185                 190
   Ser Ser Ser Ser Phe Met Met Arg Asp Ala Pro Pro Val Ala Asp Thr
           195                 200                 205
   Ser Asn His Pro Ala Ala Ala Gly Glu Arg Ala Glu Asp Val Ala Val
       210                 215                 220
   Gln Pro Gln Val Pro Leu Arg Thr Ala Leu Pro Leu Trp Met Val Ser
   225                 230                 235                 240
   His Ile Asn Gly
<210> 14
   <211> 276
   <212> PRT
   <213> hordeum vulgare
<400> 14
   Met Gly Arg Gly Lys Val Gln Leu Lys Arg Ile Glu Asn Lys Ile Asn
   1               5                   10                  15
   Arg Gln Val Thr Phe Ser Lys Arg Arg Asn Gly Leu Leu Lys Lys Ala
               20                  25                  30
   His Glu Ile Ser Val Leu Cys Asp Ala Glu Val Ala Val Ile Val Phe
           35                  40                  45
   Ser Pro Lys Gly Lys Leu Tyr Glu Tyr Ala Thr Asp Ser Ser Met Asp
       50                  55                  60
   Lys Ile Leu Glu Arg Tyr Glu Arg Tyr Ser Tyr Ala Glu Lys Ala Leu
   65                  70                  75                  80
   Ile Ser Ala Glu Ser Glu Ser Glu Gly Asn Trp Cys His Glu Tyr Arg
                   85                  90                  95
   Lys Leu Lys Ala Lys Ile Glu Thr Ile Gln Lys Cys His Lys His Leu
               100                 105                 110
   Met Gly Glu Asp Leu Asp Ser Leu Asn Leu Lys Glu Leu Gln Gln Leu
           115                 120                 125
   Glu Gln Gln Leu Glu Ser Ser Leu Lys His Ile Arg Ser Arg Lys Ser
       130                 135                 140
   His Leu Met Met Glu Ser Ile Ser Glu Leu Gln Lys Lys Glu Arg Ser
   145                 150                 155                 160
   Leu Gln Glu Glu Asn Lys Ala Leu Gln Lys Glu Leu Val Glu Arg Gln
                   165                 170                 175
   Lys Ala Ala Ser Arg Gln Gln Gln Leu Gln Gln Gln Gln Gln Gln Gln
               180                 185                 190
   Gln Met Gln Trp Glu His Gln Ala Gln Thr Gln Thr His Thr His Thr
   195 200 205
   Gln Asn Gln Pro Gln Ala Gln Thr Ser Ser Ser Ser Ser Ser Phe Met
   210 215 220
   Met Arg Asp Gln Gln Ala His Ala Pro Gln Gln Asn Ile Cys Ser Tyr
   225 230 235 240
   Pro Pro Val Thr Met Gly Gly Glu Ala Thr Ala Ala Ala Ala Ala Pro
   245 250 255
   Glu Gln Gln Ala Gln Leu Arg Ile Cys Leu Pro Pro Trp Met Leu Ser
   260 265 270
   His Leu Asn Ala
   275
<210> 15
   <211> 192
   <212> PRT
   <213> hordeum vulgare
<400> 15
   Gln Ser Met Pro Lys Thr Leu Glu Arg Tyr Gln Lys Cys Ser Tyr Gly
   1 5 10 15
   Gly Pro Asp Thr Ala Ile Gln Asn Lys Glu Asn Glu Leu Val Gln Ser
   20 25 30
   Ser Arg Asn Glu Tyr Leu Lys Leu Lys Ala Arg Val Glu Asn Leu Gln
   35 40 45
   Arg Thr Gln Arg Asn Leu Leu Gly Glu Asp Leu Gly Ser Leu Gly Ile
   50 55 60
   Lys Asp Leu Glu Gln Leu Glu Lys Gln Leu Asp Ser Ser Leu Arg His
   65 70 75 80
   Ile Arg Ser Thr Arg Thr Gln His Met Leu Asp Gln Leu Thr Asp Leu
   85 90 95
   Gln Arg Lys Glu Gln Met Leu Ser Glu Ala Asn Lys Cys Leu Arg Arg
   100 105 110
   Lys Leu Glu Glu Ser Ser Gln Gln Met Gln Gly Gln Met Trp Glu Gln
   115 120 125
   His Ala Ala Asn Leu Leu Gly Tyr Asp His Leu Arg Gln Ser Pro His
   130 135 140
   Gln Gln Gln Ala Gln His His Gly Gly Asn Gly Phe Phe His Pro Leu
   145 150 155 160
   Asp Pro Thr Thr Glu Pro Thr Leu Gln Ile Gly Tyr Thr Gln Glu Gln
   165 170 175
   Ile Asn Asn Ala Cys Val Ala Ala Ser Phe Met Pro Thr Trp Leu Pro
   180 185 190
<210> 16
   <211> 270
   <212> DNA
   <213> oriza sativa
<400> 16
   gtaagcaaga gatagggata aggggaagag gaggaagaag gaggaggtgt agggagaaac 60
   cggagcaacc tcgaagctag tccaaactag tgggaggttg tctttccggc aagccggagc 120
   ccggagctat cgatcatcaa gctttctacc ccgaccgacg aggaagaaga cgactgatca 180
   attgatcaaa ccgatctctc catagctagg tagacaggag gagaggagga agaagagggg 240
   gagaggagac ttatcttgat cgatggcgcg 270
<210> 17
   <211> 371
   <212> DNA
   <213> oriza sativa
<400> 17
   tcgacgtagg catgatggta gcagatcaac agaatgggca ttttagtaca gtcccactgt 60
   tagaagagat cactaaccca ctgactatac tgagtggcta ttctacttgt aggggctcgg 120
   agatgggcta ttccttctaa cactaataat ggcctggggg atacttgtgt tcattactag 180
   tgtaatatgg ttaataatgc ttgtgttgct gtttgctttg ctattctgat gtaccttatt 240
   tagacaagtt cccgcaggaa gtgtctttta gtattgtatt tgtcttgggc tgtggtgctt 300
   tgtttttccc taaagaactc ttgaggagct ctgttgttga accatttcaa gtaattgaga 360
   ctattgtttc c 371